# EUROPEAN PATENT SPECIFICATION

(11) **EP 1 700 908 B1**
(45) Date of publication and mention of the grant of the patent: **06.01.2016**
(21) Application number: 05077594.9
(22) Date of filing: 17.08.1999
(51) Int. Cl.: C12N 9/04, C12N 9/88, C12N 9/10, C12P 7/62

(54) **Transgenic microbial polyhydroxyalkanoate producers**
Transgene Polyhydroxyalkanoat produzierende Mikroben
Microbe transgenique pour le production de polyhydroxyalkanoate

(30) Priority: 18.08.1998 US 96852 P
(43) Date of publication of application: 13.09.2006
(62) Divisional of application: 99943723.9
(73) Proprietor: METABOLIX, INC., Cambridge, MA 02139 (US)
(72) Inventor: Huisman, Gjalt W., San Carlos, CA 94070 (US); Peoples, Oliver P., Arlington Massachusetts 02474 (US); Skraly, Frank A., Watertown Massachusetts 02472 (US)
(74) Representative: Potter Clarkson LLP

(56) References cited:
- WO-A-00/08198
- WO-A-98/04713
- WO-A-99/14313
- US-A- 5 534 432
- US-A- 5 849 894
- VALENTIN H E ET AL: "METABOLIC PATHWAY FOR BIOSYNTHESIS OF POLY(3-HYDROXYBUTYRATE-CO-4- HYDROXYBUTYRATE) FROM 4-HYDROXYBUTYRATE BY ALCALIGENES EUTROPHUS" EUROPEAN JOURNAL OF BIOCHEMISTRY, BERLIN, DE, vol. 227, no. 1/2, January 1995 (1995-01), pages 43-60, XP000867242 ISSN: 0014-2956
- HERRERO M ET AL: "TRANSPOSON VECTORS CONTAINING NON-ANTIBIOTIC RESISTANCE SELECTION MARKERS FOR CLONING AND STABLE CHROMOSOMAL INSERTION OF FOREIGN GENES IN GRAM-NETATIVE BACTERIA" JOURNAL OF BACTERIOLOGY, WASHINGTON, DC, US, vol. 172, no. 11, 1 November 1990 (1990-11-01), pages 6557-6567, XP000572232 ISSN: 0021-9193
- POIEIER Y ET AL: "PRODUCTION OF POLYHYDROXYALKANOATES, A FAMILY OF BIODEGRADABLE PLASTICS AND ELASTOMERS, IN BACTERIA AND PLANTS" BIOTECHNOLOGY, BUTTERWORTHS, LONDON, GB, vol. 13, no. 2, 13 February 1995 (1995-02-13), pages 142-150, XP000651028 ISSN: 0740-7378
- PRIETO M A ET AL: "ENGINEERING OF STABLE RECOMBINANT BACTERIA FOR PRODUCTION OF CHIRAL MEDIUM-CHAIN-LENGHT POLY-3-HYDROXYALKANOATES" APPLIED AND ENVIRONMENTAL MICROBIOLOGY, WASHINGTON,DC, US, vol. 65, no. 8, August 1999 (1999-08), pages 3265-3271, XP000870220 ISSN: 0099-2240
- FUKUI ET AL: "Cloning and analysis of the poly(3- hydroxybutyrate co-3-hydroxyhexanoate) biosynthesis genes of Aeromonas caviae" JOURNAL OF BACTERIOLOGY, AMERICAN SOCIETY FOR MICROBIOLOGY, US, vol. 179, no. 15, 1 August 1997 (1997-08-01), pages 4821-4830, XP002113257 ISSN: 0021-9193
- DENNIS D ET AL: "Formation of poly(3-hydroxybutyrate-co-3-hydroxyhexanoa te) by PHA synthase from Ralstonia eutropha" JOURNAL OF BIOTECHNOLOGY, ELSEVIER SCIENCE PUBLISHERS, AMSTERDAM, NL, vol. 64, no. 2-3, 8 October 1998 (1998-10-08), pages 177-186, XP004144017 ISSN: 0168-1656

### SEQUENCE LISTING

<110> Metabolix, Inc.
<120> Transgenic Microbial Polyhydroxyalkanoate Producers
<130> PABBE/P24111EPdiv1
<140> EP 05077594.9
   <141> 2005-11-11
<150> US 60/096,852
   <151> 1998-09-18
<160> 39
<170> PatentIn Ver. 2.1
<210> 1
   <211> 50
   <212> DNA
   <213> Artificial Sequence
<220>
   <223> Description of Artificial Sequence: oligonucleotide
<400> 1
   aagcttccca gcccgctaat gagcgggctt ttttttgaac aaaagcatgc 50
<210> 2
   <211> 71
   <212> DNA
   <213> Artificial Sequence
<220>
   <223> Description of Artificial Sequence: oligonucleotide
<400> 2
   gtctacgtac cccaggcttt acatttatgc ttccggctcg tatgttgtgt ggaattgtga 60
   gcggttcgga c                                                      71
<210> 3
   <211> 48
   <212> DNA
   <213> Artificial Sequence
<220>
   <223> Description of Artificial Sequence: oligonucleotide
<400> 3
   agcttcccag cccgctaatg agcgggcttt tttttgaaca aaagctgc 48
<210> 4
   <211> 40
   <212> DNA
   <213> Artificial Sequence
<220>
   <223> Description of Artificial Sequence: oligonucleotide
<400> 4
   cttttgttca aaaaaaagcc cgctcattag cgggctggga 40
<210> 5
   <211> 45
   <212> DNA
   <213> Artificial Sequence
<220>
   <223> Description of Artificial Sequence: oligonucleotide
<400> 5
   ggctcgtata atgtgtggag ggagaaccgc cgggctcgcg ccgtt 45
<210> 6
   <211> 53
   <212> DNA
   <213> Artificial Sequence
<220>
   <223> Description of Artificial Sequence: oligonucleotide
<400> 6
   ctagaacggc gcgagcccgg cggttctccc tccacacatt atacgagcct gca 53
<210> 7
   <211> 43
   <212> DNA
   <213> Artificial Sequence
<220>
   <223> Description of Artificial Sequence: oligonucleotide
<400> 7
   ttcagaaaat tattttaaat ttcctcttga catttatgct gca 43
<210> 8
   <211> 43
   <212> DNA
   <213> Artificial Sequence
<220>
   <223> Description of Artificial Sequence: oligonucleotide
<400> 8
   gcataaatgt caagaggaaa tttaaaataa ttttctgaat gca 43
<210> 9
   <211> 58
   <212> DNA
   <213> Artificial Sequence
<220>
   <223> Description of Artificial Sequence: oligonucleotide
<400> 9
   ctagtgccgg acccggttcc aaggccggcc gcaaggctgc cagaactgag gaagcaca 58
<210> 10
   <211> 56
   <212> DNA
   <213> Artificial Sequence
<220>
   <223> Description of Artificial Sequence: oligonucleotide
<400> 10
   tatgtgcttc ctcagttctg gcagccttgc ggccggcctt ggaaccgggt ccggca 56
<210> 11
   <211> 26
   <212> DNA
   <213> Artificial Sequence
<220>
   <223> Description of Artificial Sequence: oligonucleotide
<400> 11
   tcccctgtca taaagttgtc actgca 26
<210> 12
   <211> 26
   <212> DNA
   <213> Artificial Sequence
<220>
   <223> Description of Artificial Sequence: oligonucleotide
<400> 12
   gtgacaactt tatgacaggg gatgca 26
<210> 13
   <211> 19
   <212> DNA
   <213> Artificial Sequence
<220>
   <223> Description of Artificial Sequence: oligonucleotide
<400> 13
   tgaccaacat acgagcggc 19
<210> 14
   <211> 20
   <212> DNA
   <213> Artificial Sequence
<220>
   <223> Description of Artificial Sequence: oligonucleotide
<400> 14
   ctaccagaac tttgctttcc 20
<210> 15
   <211> 36
   <212> DNA
   <213> Artificial Sequence
<220>
   <223> Description of Artificial Sequence: oligonucleotide
<400> 15
   tgcatgcgat atcaattgtc cagccagaaa gtgagg 36
<210> 16
   <211> 20
   <212> DNA
   <213> Artificial Sequence
<220>
   <223> Description of Artificial Sequence: oligonucleotide
<400> 16
   atttattcaa caaagccgcc 20
<210> 17
   <211> 38
   <212> DNA
   <213> Artificial Sequence
<220>
   <223> Description of Artificial Sequence: oligonucleotide
<400> 17
   cccagcccgc taatgagcgg gctttttttt gaacaaaa 38
<210> 18
   <211> 44
   <212> DNA
   <213> Artificial Sequence
<220>
   <223> Description of Artificial Sequence: oligonucleotide
<400> 18
   tacgtatttt gttcaaaaaa aagcccgctc attagcgggc tggg 44
<210> 19
   <211> 63
   <212> DNA
   <213> Artificial Sequence
<220>
   <223> Description of Artificial Sequence: oligonucleotide
<400> 19
   tacgtacccc aggctttaca tttatgcttc cggctcgtat gttgtgtgga attgtgagcg 60
   gtt 63
<210> 20
   <211> 61
   <212> DNA
   <213> Artificial Sequence
<220>
   <223> Description of Artificial Sequence: oligonucleotide
<400> 20
   ttcgaaccgc tcacaattcc acacaacata cgagccggaa gcataaatgt aaagcctggg 60
   g                                                                 61
<210> 21
   <211> 14
   <212> DNA
   <213> Pseudomonas oleovorans
<400> 21
   aggaggtttt tatg 14
<210> 22
   <211> 42
   <212> DNA
   <213> Artificial Sequence
<220>
   <223> Description of Artificial Sequence: primer
<400> 22
   ggaattcagg aggttttatg gcgaccggca aaggcgcggc ag 42
<210> 23
   <211> 38
   <212> DNA
   <213> Artificial Sequence
<220>
   <223> Description of Artificial Sequence: primer
<400> 23
   gctctagaag ctttcatgcc ttggctttga cgtatcgc 38
<210> 24
   <211> 42
   <212> DNA
   <213> Artificial Sequence
<220>
   <223> Description of Artificial Sequence: primer
<400> 24
   ggaattcagg aggttttatg agtaacaaga acaacgatga gc 42
<210> 25
   <211> 38
   <212> DNA
   <213> Artificial Sequence
<220>
   <223> Description of Artificial Sequence: primer
<400> 25
   gctctagaag ctttcaacgc tcgtgaacgt aggtgccc 38
<210> 26
   <211> 42
   <212> DNA
   <213> Artificial Sequence
<220>
   <223> Description of Artificial Sequence: primer
<400> 26
   ggaattcagg aggttttatg agccaaccat cttatggccc gc 42
<210> 27
   <211> 38
   <212> DNA
   <213> Artificial Sequence
<220>
   <223> Description of Artificial Sequence: primer
<400> 27
   gctctagaag ctttcatgcg gcgtcctcct ctgttggg 38
<210> 28
   <211> 42
   <212> DNA
   <213> Artificial Sequence
<220>
   <223> Description of Artificial Sequence: primer
<400> 28
   ggaattcagg aggttttatg aggccagaaa tcgctgtact tg 42
<210> 29
   <211> 38
   <212> DNA
   <213> Artificial Sequence
<220>
   <223> Description of Artificial Sequence: primer
<400> 29
   gctctagaag ctttcagatg gcaaatgcat gctgcccc 38
<210> 30
   <211> 43
   <212> DNA
   <213> Artificial Sequence
<220>
   <223> Description of Artificial Sequence: primer
<400> 30
   aggagctcag gaggttttat gagcgcacaa tccctggaag tag 43
<210> 31
   <211> 38
   <212> DNA
   <213> Artificial Sequence
<220>
   <223> Description of Artificial Sequence: primer
<400> 31
   gctctagaag cttttaaggc agcttgacca cggcttcc 38
<210> 32
   <211> 43
   <212> DNA
   <213> Artificial Sequence
<220>
   <223> Description of Artificial Sequence: primer
<400> 32
   ggaattcagg aggttttatg acgcgtgaag tggtagtggt aag 43
<210> 33
   <211> 37
   <212> DNA
   <213> Artificial Sequence
<220>
   <223> Description of Artificial Sequence: primer
<400> 33
   gctctagaag ctttcagata cgctcgaaga tggcggc 37
<210> 34
   <211> 42
   <212> DNA
   <213> Artificial Sequence
<220>
   <223> Description of Artificial Sequence: primer
<400> 34
   ggaattcagg aggttttatg atcctcaccc cggaacaagt tg 42
<210> 35
   <211> 38
   <212> DNA
   <213> Artificial Sequence
<220>
   <223> Description of Artificial Sequence: primer
<400> 35
   gctctagaag ctttcagggc actaccttca tcgttggc 38
<210> 36
   <211> 38
   <212> DNA
   <213> Artificial Sequence
<220>
   <223> Description of Artificial Sequence: primer
<400> 36
   gctctagaag ctttcaggca gccgtcgtct tctttgcc 38
<210> 37
   <211> 39
   <212> DNA
   <213> Aeromonas caviae
<400> 37
   agaattcagg aggacgccgc atgagcgcac aatccctgg 39
<210> 38
   <211> 29
   <212> DNA
   <213> Aeromonas caviae
<400> 38
   ttcctgcagc tcaaggcagc ttgaccacg 29
<210> 39
   <211> 1680
   <212> DNA
   <213> Pseudomonas oleovorans
<400> 39
   atgagtaaca agaacaacga tgagctgcag cggcaggcct cggaaaacac cctggggctg 60
   aacccggtca tcggtatccg ccgcaaagac ctgttgagct cggcacgcac cgtgctgcgc 120
   caggccgtgc gccaaccgct gcacagcgcc aagcatgtgg cccactttgg cctggagctg 180
   aagaacgtgc tgctgggcaa gtccagcctt gccccggaaa gcgacgaccg tcgcttcaat 240
   gacccggcat ggagcaacaa cccactttac cgccgctacc tgcaaaccta tctggcctgg 300
   cgcaaggagc tgcaggactg gatcggcaac agcgacctgt cgccccagga catcagccgc 360
   ggccagttcg tcatcaacct gatgaccgaa gccatggctc cgaccaacac cctgtccaac 420
   ccggcagcag tcaaacgctt cttcgaaacc ggcggcaaga gcctgctcga tggcctgtcc 480
   aacctggcca aggacctggt caacaacggt ggcatgccca gccaggtgaa catggacgcc 540
   ttcgaggtgg gcaagaacct gggcaccagt gaaggcgccg tggtgtaccg caacgatgtg 600
   ctggagctga tccagtacaa ccccatcacc gagcaggtgc atgcccgccc gctgctggtg 660
   gtgccgccgc agatcaacaa gttctacgta ttcgacctga gcccggaaaa gagcctggca 720
   cgctactgcc tgcgctcgca gcagcagacc ttcatcatca gctggcgcaa cccgaccaaa 780
   gcccagcgcg aatggggcct gtccacctac atcgacgcgc tcaaggaggc ggtcgacgcg 840
   gtgctggcga ttaccggcag caaggacctg aacatgctcg gtgcctgctc cggcggcatc 900
   acctgcacgg cattggtcgg ccactatgcc gccctcggcg aaaacaaggt caatgccctg 960
   accctgctgg tcagcgtgct ggacaccacc atggacaacc aggtcgccct gttcgtcgac 1020
   gagcagactt tggaggccgc caagcgccac tcctaccagg ccggtgtgct cgaaggcagc 1080
   gagatggcca aggtgttcgc ctggatgcgc cccaacgacc tgatctggaa ctactgggtc 1140
   aacaactacc tgctcggcaa cgagccgccg gtgttcgaca tcctgttctg gaacaacgac 1200
   accacgcgcc tgccggccgc cttccacggc gacctgatcg aaatgttcaa gagcaacccg 1260
   ctgacccgcc cggacgccct ggaggtttgc ggcactccga tcgacctgaa acaggtcaaa 1320
   tgcgacatct acagccttgc cggcaccaac gaccacatca ccccgtggca gtcatgctac 1380
   cgctcggcgc acctgttcgg cggcaagatc gagttcgtgc tgtccaacag cggccacatc 1440
   cagagcatcc tcaacccgcc aggcaacccc aaggcgcgct tcatgaccgg tgccgatcgc 1500
   ccgggtgacc cggtggcctg gcaggaaaac gccaccaagc atgccgactc ctggtggctg 1560
   cactggcaaa gctggctggg cgagcgtgcc ggcgagctga aaaaggcgcc gacccgcctg 1620
   ggcaaccgtg cctatgccgc tggcgaggca tccccgggca cctacgttca cgagcgttga 1680

## Description

### Background Of The Invention

The present invention is generally in the field of biosynthesis of poly(3-hydroxyallcanoates), and more particularly to improved microbial strains useful in commercial production of polyhydroxyalkanoates.

Poly(3-hydroxyalkanoates) (PHAs) are biological polyesters synthesized by a broad range of bacteria. These polymers are biodegradable and biocompatible thermoplastic materials, produced from renewable resources, with a broad range of industrial and biomedical applications (Williams & Peoples, CHEMTECH 26:38-44 (1996)). PHA, biopolymers have emerged from what was originally considered to be a single homopolymer, poly-3-hydroxybutyrate (PHB) into a broad class of polyesters with different monomer compositions and a wide range of physical properties. About 100 different monomers have been incorporated into the PHA polymers (Steinbuchel & Valentin, FEMS Microbiol. Lett. 128:219-28 (1995)).

It has been useful to divide the PHAs into two groups according to the length of their side chains and their biosynthetic pathways. Those with short side chains, such as PHB, a homopolymer of *R*-3-hydroxybutyric acid units, are crystalline thermoplastics, whereas PHAs with long side chains are more elastomeric. The former have been known for about seventy years (Lemoigne & Roukhehnan, 1925), whereas the latter materials were discovered relatively recently (deSmet et al., J. Bacteriol. 154:870-78 (1983)). Before this designation, however, PHAs of microbial origin containing both (*R*)-3-hydroxybutyric acid units and longer side chain (*R*)-3-hydroxyacid units from C₅ to C₁₆ had been identified (Wallen & Rohweder, Environ. Sci. Technol. 8:576-79 (1974)). A number of bacteria which produce copolymers of (*R*)-3-hydroxybutyric acid and one or more long side chain hydroxyacid units containing from five to sixteen carbon atoms have been identified (Steinbuchel & Wiese, Appl. Microbiol. Biotechnol. 37:691-97 (1992); Valentin et al., Appl. Microbiol. Biotechnol. 36:507-14 (1992); Valentin et al., Appl. Microbiol. Biotechnol. 40:710-16 (1994); Abe et al., Int. J. Biol. Macromol. 16:115-19 (1994); Lee et al., Appl. Microbiol. Biotechnol. 42:901-09 (1995); Kato et al., Appl. Microbiol. Biotechnol. 45:363-70 (1996); Valentin et al., Appl. Microbiol. Biotechnol. 46:261-67 (1996); U.S. Patent No. 4,876,331 to Doi). A combination of the two biosynthetic pathways outlined described above provide the hydroxyacid monomers. These copolymers can be referred to as PHB-co-HX (where X is a 3-hydroxyallcanoate or alkanoate or alkenoate of 6 or more carbons). A useful example of specific two-component copolymers is PHB-co-3-hydroxyhexanoate (PHB-co-3HH) (Brandl et al., Int. J. Biol. Macromol. 11:49-55 (1989); Amos & McInerey, Arch. Microbiol. 155:103-06 (1991); U.S. Patent No. 5,292,860 to Shiotani *et al*.).

PHA production by many of the microorganisms in these references is not commercially useful because of the complexity of the growth medium, the lengthy fermentation processes, or the difficulty of down-stream processing of the particular bacterial strain. Genetically engineered PHA production systems with fast growing organisms such as *Escherichia coli* have been developed. Genetic engineering also allows for the improvement of wild type PHA production microbes to improve the production of specific copolymers or to introduce the capability to produce different PHA polymers by adding PHA biosynthetic enzymes having different substrate-specificity or even kinetic properties to the natural system. Examples of these types of systems are described in Steinbuchel & Valentin, FEMS Microbiol Lett. 128:219-28 (1995). PCT WO 98/04713 describes methods for controlling the molecular weight using genetic engineering to control the level of the PHA synthase enzyme. Commercially useful strains, including *Alcaligenes eutrophus* (renamed as *Ralstonia eutropha), Alcaligenes latus, Azotobacter vinlandii,* and *Pseudomonads,* for producing PHAs are disclosed in Lee, Biotechnology & Bioengineering 49:1-14 (1996) and Braunegg et al., (1998), J. Biotechnology 65:127-161.

The development of recombinant PHA production strains has followed two parallel paths. In one case, the strains have been developed to produce copolymers, a number of which have been produced in recombinant *E. coli.* These copolymers include poly(3-hydroxybutyrate-co-3-hydroxyvalerate) (PHBV), poly(3-hydroxybutyrate-co-4-hydroxybutyrate) (P3HB-co-4HB), poly(4-hydroxybutyrate) (P4HB) and long side chain PHAs comprising 3-hydroxyoctanoate units (Madison and Huisman, 1999. Strains of *E. coli* containing the *phb* genes on a plasmid have been developed to produce P(3HB-3HV) (Slater, et al., Appl. Environ. Microbiol. 58:1089-94 (1992); Fidler & Dennis, FEMS Microbiol. Rev. 103:231-36 (1992); Rhie & Dennis, Appl. Environ. Micobiol. 61:2487-92 (1995); Zhang, H. et al., Appl. Environ. Microbiol. 60:1198-205 (1994)). The production of P(4HB) and P(3HB-4HB) in *E. coli* is achieved by introducing genes from a metabolically unrelated pathway into a P(3HB) producer (Hein, et al., FEMS Microbiol. Lett. 153:411-18 (1997); Valentin & Dennis, J. Biotechnol. 58:33-38 (1997)). *E. coli* also has been engineered to produce medium short chain polyhydroayalkanoates (msc-PHAs) by introducing the *phaCl* and *phaC2* gene of *P. aeruginosa* in a *fadB*::kan mutant (Langenbach, et al., FEMS Microbiol. Lett. 150:303-09 (1997); Qi, et al., FEMS Microbiol. Lett. 157:155-62 (1997)).

Although studies demonstrated that expression of the *A. eutrophus* PHB biosynthetic genes encoding PHB polymerase, -ketothiolase, and acetoacetyl-CoA reductase in *E. coli* resulted in the production of PHB (Slater, et al., J. Bacteriol. 170:4431-36 (1988); Peoples & Sinskey, J. Biol. Chem. 264:15298-303 (1989); Schubert, et al., J. Bacteriol. 170:5837-47 (1988)), these results were obtained using basic cloning plasmid vectors and the systems are unsuitable for commercial production since these strains lacked the ability to accumulate levels equivalent to the natural producers in industrial media.

For commercial production, these strains have to be made suitable for large scale fermentation in low cost industrial medium. The first report of recombinant P(3HB) production experiments in fed-batch cultures used an expensive complex medium, producing P(3HB) to 90 g/L in 42 hours using a pH-stat controlled system (Kim, et al., Biotechnol. Lett. 14:811-16 (1992)). Using stabilized plasmids derived from either medium- or high-copy-number plasmids, it was shown that *E.* coli XL1-Blue with the latter type plasmid is required for substantial P(3HB) accumulation (Lee, et al., Ann. N.Y. Acad. Sci. 721:43-53 (1994)). In a fed-batch fermentation on 2% glucose/LB medium, this strain produced 81% P(3HB) at a productivity of 2.1 g/L-hr (Lee, et al., J. Biotechnol. 32:203-11 (1994)). The P(3HB) productivity was reduced to 0.46 g/L-hr in minimal medium, but could be recovered by the addition of complex nitrogen sources such as yeast extract, tryptone, casamino acids, and collagen hydrolysate (Lee & Chang, Adv. Biochem. Eng. Biotechnol. 52:27-58 (1995); Lee, et al., J. Ferment. Bioeng. 79:177-80 (1995)).

Although recombinant *E. coli* XL1-blue is able to synthesize substantial levels of P(3HB), growth is impaired by dramatic filamentation of the cells, especially in defined medium (Lee, et al., Biotechnol. Bioeng. 44:1337-47 (1994); Lee, Biotechnol. Lett. 16:1247-52 (1994); Wang & Lee, Appl. Environ. Microbiol. 63:4765-69 (1997)). By overexpression of FtsZ in this strain, biomass production was improved by 20% and P(3HB) levels were doubled (Lee & Lee, J. Environ. Polymer Degrad. 4:131-34 (1996)). This recombinant strain produced 104 g/L P(3HB) in defined medium corresponding to 70% of the cell dry weight. The volumetric productivity of 2 g/L-hr, however, is lower than achievable with *R. eutropha.* Furthermore, about 15% of the cells lost their ability to produce PHB by the end of the fermentation (Wang & Lee, Biotechnol. Bioeng. 58:325-28 (1998)).

Recombinant *E. coli* P(3HB-3HV) producers reportedly are unable to grow to a high density and therefore are unsuited for commercial processes (Yim, et al., Biotechnol. Bioeng. 49:495-503 (1996)). In an attempt to improve P(3HB-3HV) production in a recombinant strain, four *E. coli* strains (XL1-Blue, JM109, HB101, and DH5α) were tested by Yim *et al.* All four recombinant *E. coli* strains synthesized P(3HB-3HV) when grown on glucose and propionate with HV fractions of 7% (Yim, et al., Biotechnol. Bioeng. 49:495-503 (1996)). Unlike other strains studied (Slater, et al., Appl. Environ. Microbiol. 58:1089-94 (1992)), recombinant XL1-Blue incorporates less than 10% HV when the propionic acid concentration is varied between 0 and 80 mM. HV incorporation and PHA formation were increased by pre-growing cells on acetate followed by glucose/propionate addition at a cell density of around 10⁸ cells per ml. Oleate supplementation also stimulated HV incorporation. This recombinant XL1-Blue when pregrown on acetate and with oleate supplementation reached a cell density of 8 g/L, 75% of which was P(3HB-3HV) with an HV fraction of 0.16 (Yim, et al., Biotechnol. Bioeng. 49:495-503 (1996)).

One of the challenges of producing P(3HB) in recombinant organisms is the stable and constant expression of *the phb* genes during fermentation. Often P(3HB) production by recombinant organisms is hampered by the loss of plasmid from the majority of the bacterial population. Such stability problems may be attributed to the metabolic load exerted by the need to replicate the plasmid and synthesize P(3HB), which diverts acetyl-CoA to P(3HB) rather than to biomass. In addition, plasmid copy numbers often decrease upon continued fermentation because only a few copies provide the required antibiotic resistance or prevent cell death by maintaining *parB.* For these reasons, a runaway plasmid was designed to suppress the copy number of the plasmid at 30 C and induce plasmid replication by shifting the temperature to 38 C (Kidwell, et al., Appl. Environ. Microbial. 61:1391-98 (1995)). Using this system, P(3HB) was produced to about 43% of the cell dry weight within 15 hours after induction with a volumetric production of 1 gram P(3HB) per liter per hour. Although this productivity is of the same order of magnitude as natural P(3HB) producers, strains harboring these *parB*-stabilized runaway replicons still lost the capacity to accumulate P(3HB) during prolonged fermentations.

While the instability of the *phb* genes in high cell-density fermentations affects the PHA cost by decreasing the cellular P(3HB) yields, the cost of the feedstock also contributes to the comparatively high price of PHAs. The most common substrate used for P(3HB) production is glucose. Consequently, *E. coli* and *Klebsiella* strains have been examined for P(3HB) formation on molasses, which cost 33-50% less than glucose (Zhang, et al., Appl. Environ, Microbiol. 60:1198-1205 (1994)). The main carbon source in molasses is sucrose. Recombinant *E. coli* and *K. aerogenes* strains carrying the *phb* locus on a plasmid grown in minimal medium with 6% sugarcane molasses accumulated P(3HB) to approximately 3 g/L corresponding to 45% of the cell dry weight. When the *K. aerogenes* was grown fed-batch in a 10 L fermenter on molasses as the sole carbon source, P(3HB) was accumulated to 70% its cell dry weight, which corresponded to 24 g/L. Although the *phb* plasmid in *K. aerogenes* was unstable, this strain shows promise as a P(3HB) producer on molasses, especially since *fadR* mutants incorporate 3HV up to 55% in the presence of propionate (Zhang, et al., Appl. Environ. Microbiol. 60:1198-1205 (1994)).

U.S. Patent No. 5,334,520 to Dennis discloses the production of PHB in *E. coli* transformed with a plasmid containing the *phbCAB* genes. A *rec*⁻, *lac*⁺ *E. coli* strain was grown on whey and reportedly accumulates PHB to 85% of its cell dry weight. U.S. Patent No. 5,371,002 to Dennis et al. discloses methods to produce PHA in recombinant *E. coli* using a high copy number plasmid vector with *phb* genes in a host that expresses the acetate genes either by induction, constitutively, or from a plasmid. U. S. Patent No. 5,512,456 to Dennis discloses a method for production and recovery of PHB from transformed *E. coli* strains. These *E. coli* strains are equipped with a vector containing the *phb* genes and a vector containing a lysozyme gene. High copy number plasmids or runaway replicons are used to improve productivity. The vectors are stabilized by *parB* or by *supF*/*dnaB*(*am*). Using such strains, a productivity of 1.7 g/L-hr was obtained corresponding to 46 g/L PHB in 25 hrs, after which the plasmid was increasingly lost by the microbial population. PCT WO94/21810 discloses the production of PHB in recombinant strains of *E*. *coli* and *Klebsiella aerogenes* with sucrose as a carbon source. PCT WO 95/21257 discloses the improved production of PHB in transformed prokaryotic hosts. Improvements in the transcription regulating sequences and ribosome binding site improve PHB formation by the plasmid based *phb* genes. The plasmid is stabilized by the *parB* locus. PHB production by this construct is doubled by including the 361 nucleotides that are found upstream of *phbC* in *R. eutropha* instead of only 78 nucleotides. It is generally believed that PHB production by recombinant microorganisms requires high levels of expression using stabilized plasmids. Since plasmids are available in the cell in multiple copies, ranging from one to several hundreds, the use of plasmids ensured the presence of multiple copies of the genes of interest. Since plasmids may be lost, stabilization functions are introduced. Such systems, which are described above, have been tested for PHB production, and the utility of these systems in industrial fermentation processes has been investigated. However, overall PHB yield is still affected by loss of *phb* genes.

It is therefore an object of the present invention to provide recombinant microorganisms strains useful in industrial fermentation processes which can accumulate commercially significant levels of PHB while providing stable and constant expression of the *phb* genes during fermentation.

It is another object of the present invention to provide transgenic microbial strains for enhanced production of poly(3-hydroxyalkanoates).

It is another object of the present invention to provide transgenic microbial strains which yield stable and constant expression of *the phb* genes during fermentation and accumulate commercially significant levels of PHB, and methods of use thereof.

### Summary Of The Invention

Accordingly, a first aspect of the present invention provides a genetically engineered microorganism having a PHA synthase gene involved in synthesis of polyhydroxyalkanoates, wherein the microorganism is transformed with a *phaJ* gene encoding enoyl-CoA hydratase, and either or both of the PHA synthase or *phaJ* genes are integrated into the chromosome of the microorganism, and the microorganism produces polyhydroxyalkanoate, wherein the microorganism is selected from the group consisting of *E*. *coli, Alcaligenes latus, Alcaligenese eutrophus, Azotobacter, Pseudomonas putida*, and *Ralstonia eutropha...*

In one embodiment, the *phaJ* gene may be integrated into the chromosome of the bacterium and may, for example, be inserted using transposon mutagenesis.

In another embodiment, the *phaJ* gene may be present within the microorganism on a plasmid.

It may be preferred that the *phaJ* gene encodes the (R)-specific enoyl-CoA hydratase from *A. caviae* (J12).

Optionally, the PHA synthase gene may be a transgene. Optionally, the PHA synthase gene may be integrated into the chromosome of the bacterium. Optionally, the PHA synthase gene may be a PHB synthase gene. It may be preferred that the PHA synthase is PHB polymerase from *R. eutropha* (C1), PHA polymerase from *P. oleovorans* (C3), or PHB polymerase from *A. caviae* (C12).

In one embodiment, the microorganism may comprising multiple genes involved in synthesis of polyhydroxyalkanoate wherein the genes are integrated operably linked as an operon.

Optionally, the PHA synthase and/or the *phaJ* gene may be integrated operably linked under the control of a promoter and/or integrated operably linked with upstream activating sequences, an mRNA stabilizing sequence, or a selection marker, and/or operably linked with promoter including a consensus *E*. *coli* pho box and -35 promoter region that is regulated by the phosphate concentration in the medium, preferably wherein the promoter is selected from the group consisting of promoter that induces expression under general stress conditions such as nutrient limitation, pH or heat shock, and administration of toxic chemicals.

Optionally, the PHA synthase and/or the *phaJ* gene may be isolated or derived from a microorganism selected from the group consisting of *A. eutrophus, Aeromonas caviae, Zoogloea ramigera, Nocardia, Rhodococcus, Pseudomonas Sp. 61-3, Pseudomonas acidophila, Pseudomonas oleovarans, Chromobacterium violaceum,* and *Alcaligenes latus.*

Optionally, the PHA synthase and/or the *phaJ* gene may be integrated as a single copy on the chromosome of the microorganism.

In a preferred embodiment, a genetically engineered microorganism of the first aspect of the present invention is capable of producing PHA from fatty acids.

Optionally, the genetically engineered microorganism of the first aspect of the present invention may further have at least one gene involved in synthesis of polyhydroxyalkanoates selected from the group consisting of thiolase, reductase, and acyl-CoA transferase, integrated into the chromosome.

A second aspect of the present invention provides a method for producing polyhydroxyalkanoates comprising culturing genetically engineered micro-organisms as defined by the first aspect of the present invention, with appropriate substrate under conditions wherein the micro-organisms produce polyhydroxyalkanoate. Preferably, the substrate includes a fatty acid, such as octanoate and/or oleate.

A third aspect of the present invention provides for the use of an isolated DNA molecule comprising a sequence encoding enoyl-CoA hydratase (*PhaJ*) to modify the type of substrate used in the production of PHA, or to enhance the level, or cause a shift in the type, of PHA produced, by a PHA-producing micro-organism by integration of the DNA sequence into the chromosome of the PHA-producing micro-organism compared to the level, or type, of PHA produced by the same micro-organism prior to the integration of the DNA molecule. Preferably, the use is to cause a production of PHA from fatty acids.

Transgenic microbial strains are described herein which contain the genes required for PHA formation integrated on the chromosome. The strains are advantageous in PHA production processes, because (1) no plasmids need to be maintained, generally obviating the required use of antibiotics or other stabilizing pressures, and (2) no plasmid loss occurs, thereby stabilizing the number of gene copies per cell throughout the fermentation process, resulting in homogeneous PHA product formation throughout the production process. Genes are integrated using standard techniques, preferably transposon mutagenesis. In a preferred embodiment wherein mutiple genes are incorporated, these are incorporated as an operon. Sequences are used to stabilize mRNA, to induce expression as a function of culture conditions (such as phosphate concentration), temperature, and stress, and to aid in selection, through the incorporation of selection markers such as markers conferring antibiotic resistance.

### Brief Description Of The Drawings

Figure 1 is a diagram showing the construction of pMNXTp₁kan, pMNXTp₁cat, pMSXTp₁kan, and pMSXTp₁cat.
Figure 2 is a diagram showing the construction of pMUXC₅cat.
Figure 3 is a diagram showing the construction of pMUXAB₅cat, pMUXTp₁AB₅kan, pMUXTp₁₁AB₅kan, pMUXTp₁₂AB₅kan, and pMUXTp₁₃AB₅kan.

### Detailed Description Of The Invention

By randomly inserting genes that encode PHA biosynthetic enzymes into the chromosome of *E. coli*, means have been identified to directly achieve high levels of expression from strong endogenous promoters at sites that are non-essential for growth of the host in industrial medium based fermentations. As demonstratd by the examples, *E. coli* strains have been obtained using these techniques that produce PHAs in levels exceeding 85% of the cell dry weight from single copy genes on the chromosome. Expression of the *phb* genes in these strains is not dependent on the upstream sequences of *phbC* in *R. eutropha* nor on a high copy number construct. Maintenance of the *phb* genes by these strains is independent of the supplementation of antibiotics, the presence of stabilizing loci such as *parB* or *hok*/*sok* or any other selective pressure. The ultra-high level of expression required in the plasmid-based systems has been found to be completely unnecessary. Furthermore, unlike the most successful fermentations reported to date (Wang & Lee, Biotechnol. Bioeng. 58:325-28 (1998)) for recombinant plasmid-based *E. coli*, fermentation with these strains provides that virtually all of the cells contain PHB at the end of the fermentation.

Despite the low copy number, these transgenic bacteria accumulate PHB to levels observed for wild-type organisms. The host used for recombinant PHB production also is an important parameter in designing a plasmid-based *E. coli* system. For example, although W3110 strains were poor PHB producers when using a plasmid-based system, it was found that by integrating *the phb* genes into the chromosome of this same host, the host retained excellent growth characteristics while accumulating commercially significant levels of PHB.

### Methods and Materials for Producing the Microbial Strains

### Bacterial Strains to be Modified

A number of bacteria can be genetically engineered to produce polyhydroxyalkanoates. These include organisms that already produce polyhydroxyalkanoates, modified to utilize alternative substrates or incorporate additional monomers, or to increase production, and organisms that do not produce polyhydroxyalkanoates, but which expresses none to some of the enzymes required for production of polyhydroxylkanoates. Examples include *E. coli, Alcaligenes latus, Alcaligenese eutrophus*, *Azotobacter, Pseudomonas putida, and Ralstonia eutropha*.

### Methods for Venerating Transgenic PHB Producers

Methods for incorporating engineered gene constructs into the chromosomal DNA of bacterial cells are well known to those skilled in the art. Typical integration mechanisms include homologous recombination using linearized DNA in *recBC* or *recD* strains followed by P1 transduction (Miller 1992, A Short Course in Bacterial Genetics: A laboratory manual & Handbook for Escherichia coli and Related Bacteria. Cold Spring Harbor laboratory Press, Cold Spring Harbor, New York) special plasmids (Hamilton et al., J. Bacteriol. 171:4617 (1989); Metcalf et al., Plasmid 35:1 (1996); U.S. Patent No. 5,470,727 to Mascarenhas et al.), or by random insertion using transposon based systems (Herrero et al. J. Bacteriol. 172:6557 (1990); Peredelchuk & Bennett, Gene 187:231 (1997); U.S. Patent No. 5,595,889 to Richaud et al.; U.S. Patent No. 5,102,797 to Tucker et al.). In general, the microbial strains containing an insertion are selected on the basis of an acquired antibiotic resistance gene that is supplied by the integrated construct. However, complementation of auxotrophic mutants can also be used.

Expression of the genes of interest for chromosomal integration can be achieved by including a transcription activating sequence (promoter) in the DNA construct to be integrated. Site-directed, homologous recombination can be combined with amplification of expression of the genes of interest, as described by U.S. patent No. 5,00,000 to Ingram et al. Although mini-transposon systems have been used for a number of years, they have been designed such that the expression level of the integrated gene of interest is not modulated. Ingram, et al. selected for increased expression of a foreign gene inserted into the *E. coli* chromosome by homologous recombination. This was achieved by inserting a promoter-less chloroamphenicol (Cm) resistance gene downstream of the gene of interest to create a transcriptional fusion. After a transcriptional fusion of the alcohol dehydrogenase gene with a promoterless chloramphenicol acetyl transferase genes is integrated in the *pfl* gene, increased expression is achieved by selecting mutants on increasing concentrations of chloramphenicol. However, in chemostat studies these stabilzed strains still lost the capacity to produce ethanol (Lawford & Rousseau, Appl. Biochem. Biotechnol. 57-58:293-305 (1996)). Also, strains that contained the ethanologenic genes on the chromosome demonstrated a decreased growth rate in glucose minimal medium (Lawford & Rousseau, Appl. Biochem. Biotechnol., 57-58:277-92 (1996)).

These approaches have been combined and modified to randomly integrate a mini-transposon into the chromosome to select for healthy, fast growing transgenic strains coupled with a screening system for modulating expression of the integrated genes. A series of expression cassettes have been developed for inserting heterologous genes into bacterial chromosomes. These cassettes are based on the transposon delivery systems described by Herrero et al., J. Bacteriol. 172:6557-67 (1990); de Lorenzo et al., J. Bacteriol. 172:6568 (1990). Although these systems specify RP4-mediated conjugal transfer and use only transposon Tn10 and Tn5, any combination of transposon ends and delivery system could be adapted for the technology described, resulting in sustained and homogeneous PHA production.

The following general approach is used for generating transgenic *E. coli* PHB producers: (1) a promoterless antibiotic resistance (*abr*) gene is cloned in the polylinker of a suitable plasmid such as pUC18NotI or pUC18SfiI so that the major part of the polylinker is upstream of *abr*; (2) *phb* genes are subsequently cloned upstream of and in the same orientation as the *abr* gene; (3) the *phb-abr* cassette is excised as a *NotI* or *AvrII* fragment (*AvrII* recognizes the *SfiI* site in pUC18SfiI) and cloned in the corresponding sites of any plasmid like those from the pUT- or pLOF-series; (4) the resulting plasmids are maintained in *E. coli* λpir strains and electroporated or conjugated into the *E. coli* strain of choice in which these plasmids do not replicate; and (5) new strains in which the *phb-abr* cassette has successfully integrated in the chromosome are selected on selective medium for the host (e.g., naladixic acid when the host is naladixic acid resistant) and for the cassette (e.g., chloramphenicol, kanamycin, tetracyclin, mercury chloride, bialaphos). The resulting *phb* integrants are screened on minimal medium in the presence of glucose for growth and PHB formation.

Several modifications of this procedure can be made. If the promotorless antibiotic resistance marker is not used, the insertion of the PHA genes is selected based on a marker present in the vector and integrated strains producing the desired level of PHA are detected by screening for PHA production. The *phb* genes may have, but do not need, endogeneous transcription sequences, such as upstream activating sequences, RNA polymerase binding site, and/or operator sequences. If the *phb* genes do not have such sequences, the described approach is limited to the use of vectors like the pUT series in which transcription can proceed through the insertion sequences. This limitation is due to the inability of RNA polymerase to read through the Tn10 flanking regions of the pLOF plasmids. The *abr* gene may carry its own expression sequences if so desired. Instead of an *abr* gene, the construct may be designed such that an essential gene serves as selective marker when the host strain has a mutation in the corresponding wild-type gene. Examples of genes useful for this purpose are generally known in the art. Different constructs can be integrated into one host, either subsequently or simultaneously, as long as both constructs carry different marker genes. Using multiple integration events, *phb* genes can be integrated separately, e.g., the PHB polymerase gene is integrated first as a *phbC-cat* cassette, followed by integration of the thiolase and reductase genes as a *phbAB-kan* cassette. Alternatively, one cassette may contain all *phb* genes whereas another cassette contains only some *phb* genes required to produce a desired PHA polymer.

In some cases a transposon integration vector such as pJMS 11 (Panke et al. Appl. Enviro. Microbiol. 64: 748-751) may be used such that the selectable marker can be excised from the chromosome of the integrated strain. This is useful for a number of reasons including providing a mechanism to insert multiple transposon constructs using the same marker gene by excising the marker following each insertion event.

### Sources of phb and Other Genes Involved in PHA Formation

A general reference is Madison and Huisman, 1999, Microbiology and Molecular Biology Reviews 63: 21-53. The *phb* genes may be derived from different sources and combined in a single organism, or from the same source.

### Thiolase Encoding Genes

Thiolase encoding genes have been isolated from *Alcaligenes latus, Ralstonia eutropha* (Peoples & Sinskey, J. Biol. Chem. 264(26):15298-303 (1989*); Acinetobacter* sp. (Schembri, et al., J.Bacteriol. 177(15):4501-7 (1995)), *Chromotium vinosum* (Liebergesell & Steinbuchel, Eur. J. Biochem. 209(1):135-50 (1992)), *Pseudomonas acidophila, Pseudomonas denitrificans* (Yabutani, et al., FEMS Microbiol. Lett. 133 (1-2):85-90 (1995)), *Rhizobium meliloti* (Tombolini, et al., Microbiology 141:2553-59 (1995)), *Thiocystis violacea* (Liebergesell & Steinbuchel, Appl. Microbiol. Biotechnol. 38(4):493-501 (1993)), and *Zoogloea ramigera* (Peoples, et al., J. Biol. Chem. 262(1):97-102 (1987)).

Other genes that have not been implicated in PHA formation but which share significant homology with the *phb* genes and/or the corresponding gene products may be used as well. Genes encoding thiolase-and reductase- like enzymes have been identified in a broad range of non-PHB producing bacteria. *E. coli* (U29581, D90851, D90777), *Haemophilus influenzae* (U32761), *Pseudomonas fragi* (D10390), *Pseudomonas aeruginosa* (U88653), *Clostridium acetobutylicum* (U08465), *Mycobacterium leprae* (U00014), *Mycobacterium tuberculosis* (Z73902), *Helicobacter pylori* (AE000582), *Thermoanaerobacterium thermosaccharolyticum* (Z92974), *Archaeoglobus fulgidus* (AE001021), *Fusobacterium nucleatum* (U37723), *Acinetobacter calcoaceticus* (L05770), *Bacillus subtilis* (D84432, Z99120, U29084), and *Synechocystis* sp. (D90910) all encode one or more thiolases from their chromosome. Eukaryotic organisms such as *Saccharomyces cerevisiae* (L20428), *Schizosaccharomyces pombe* (D89184), *Candida tropicalis* (D13470), *Caenorhabditis elegans* (U41105), human (S70154), rat (D13921), mouse (M35797), radish (X78116), pumpkin (D70895), and cucumber (X67696) also express proteins with significant homology to the 3-ketothiolase from *R*. *eutropha.*

### Reductase Encoding Genes

Reductase encoding genes have been isolated from *A. latus, R. eutropha* (Peoples & Sinskey, J. Biol. Chem. 264(26):15298-303 (1989); *Acinetobacter* sp. (Schembri, et al., J.Bacteriol. 177(15):4501-7 (1995)), C. *vinosum* (Liebergesell & Steinbuchel, Eur. J. Biochem. 209(1):135-50 (1992)), *P. acidophila, P. denitrificans* (Yabutani, et al., FEMS Microbiol. Lett. 133 (1-2):85-90 (1995)), *R*. *meliloti* (Tombolini, et al., Microbiology 141:2553-59 (1995)), and Z. *ramigera* (Peoples, et al., J. Biol. Chem. 262(1):97-102 (1987)).

Other genes that have not been implicated in PHA formation but which share significant homology with *the phb* genes and/or the corresponding gene products may be used as well. Genes with significant homology to the *phbB* gene encoding acetoacetyl CoA reductase have been isolated from several organisms, including *Azospirillum brasiliense* (X64772, X52913) *Rhizobium* sp. (U53327, Y00604), *E. coli* (D90745), *Vibrio harveyi* (U39441), *H. influenza* (U32701), *B. subtilis* (U59433), *P. aeruginosa* (U91631), *Synechocystis* sp. (D90907), *H. pylori* (AE000570), *Arabidopsis thaliana* (X64464), *Cuphea lanceolata* (X64566) and *Mycobacterium smegmatis* (U66800).

### PHA Polymerase Encoding Genes

PHA polymerase encoding genes have been isolated from *Aeromonas caviae* (Fukui & Doi, J. Bacteriol. 179(15):4821-30 (1997)), *A. lotus, R. eutropha* (Peoples & Sinskey, J. Biol. Chem 264(26):15298-303 (1989); *Acinetobacter* (Schembri, et al., J.Bacteriol. 177(15):4501-7 (1995)), C. *vinosum* (Liebergesell & Steinbuchel, Eur. J. Biochem. 209(1):135-50 (1992)), *Methylobacterium extorquens* (Valentin & Steinbuchel, Appl. Microbiol. Biotechnol. 39(3):309-17 (1993)), *Nocardia corallina* (GenBank Acc. No. AF019964), *Nocardia salmonicolor, P. acidophila, P. denitrificans* (Ueda, et al., J. Bacteriol. 178(3):774-79 (1996)), *Pseudomonas aeruginosa* (Timm & Steinbuchel, Eur. J. Biochem. 209(1):15-30 (1992)), *Pseudomonas oleovorans* (Huisman, et al., J. Biol. Chem. 266:2191-98 (1991)), *Rhizobium etli* (Cevallos, et al., J. Bacteriol. 178(6):1646-54 (1996)), *R*. *meliloti* (Tombolini, et al., Microbiology 141 (Pt 10):2553-59 (1995)), *Rhodococcus ruber* (Pieper & Steinbuchel, FEMS Microbiol. Lett. 96(1):73-80 (1992)), *Rhodospirrilum rubrum* (Hustede, et al., FEMS Microbiol. Lett. 93:285-90 (1992)), *Rhodobacter sphaeroides* (Steinbuchel, et al., FEMS Microbiol. Rev. 9(2-4):217-30 (1992); Hustede, et al., Biotechnol. Lett. 15:709-14 (1993)), *Synechocystis* sp. (Kaneko, DNA Res. 3(3):109-36 (1996)), *T. violaceae* (Liebergesell & Steinbuchel, Appl. Microbiol. Biotechnol. 38(4):493-501 (1993)), and *Z*. *ramigera* (GenBank Acc. No. U66242).

Vectors for Incorporation of Genes into the Bacterial Chromosomes The pUT and pLOF series of plasmid transposon delivery vectors useful in the PHA-producing methods described herein use the characteristics of transposon Tn5 and transposon Tn10, respectively. The transposase genes encoding the enzymes that facilitate transposition are positioned outside of the 'transposase recognition sequences' and are consequently lost upon transposition. Both Tn5 and Tn10 are known to integrate randomly in the target genome, unlike, for example, the Tn7 transposon. However, generally any transposon can be modified to facilitate the insertion of heterologous genes, such as *the phb* genes, into bacterial genomes. This methodology thus is not restricted to the vectors used in the methods described herein.

### Methods and Materials for Screening for Enhanced Polymer Production

### Screening of Bacterial Strains

The technology described above allows for the generation of new PHA producing strains and also provides new bacterial strains that are useful for screening purposes. Table 1 below shows the different combinations of chromosomally and plasmid encoded PHB enzymes and how specific strains can be used to identify new or improved enzymes.

Besides a screening tool for genes that express improved enzymes, *E. coli* strains with a complete PHA pathway integrated on the chromosome can be used to screen for heterologous genes that affect PHA formation. *E. coli* is a useful host because genes are easily expressed from a multitude of plasmid vectors: high copy-number, low copy-number, chemical or heat inducible, etc. and mutagenesis procedures have been well established for this bacterium. In addition, the completely determined genomic sequence of *E. coli* facilitates the characterization of genes that affect PHA metabolism.

Transgenic *E. coli* strains expressing an incomplete PHA pathway can be transformed with gene libraries to identify homologs of the missing gene from other organisms, either prokaryotic or eukaryotic. Because these screening strains do not have the complete PHA biosynthetic pathway, the missing functions can be complemented and identified by the ability of the host strain to synthesize PHA. Generally PHA synthesizing bacterial colonies are opaque on agar plates, whereas colonies that do not synthesize PHB appear translucent. Clones from a gene library that complement the missing gene confer a white phenotype to the host when grown on screening media. Generally screening media contains all essential nutrients with excess carbon source and an antibiotic for which resistance is specified by the vector used in the library construction.

Besides new genes, genes encoding improved PHA biosynthetic enzymes can also be screened for. A mutagenized collection of plasmids containing a *phb* biosynthetic gene into an *E. coli* host strain lacking this activity but containing genes encoding the other PHA biosynthetic enzymes can be screened for increased or altered activity. For example, PHA polymerases with increased activity can be screened for in a strain that expresses thiolase and reductase from the chromosome by identifying PHB-containing colonies under conditions that support PHB formation poorly. mcl-PHA polymerases with an increased specificity towards C₄ can similarly be screened for under PHB accumulation promoting conditions. Altered activities in the *phaG* encoded ACP::CoA transferase can be screened for by expressing mutated versions of this gene in a *phbC* integrant and screening for PHB formation from short chain fatty acids. Enzymes that have increased activity under sub-optimal physical conditions (e.g., temperature, pH, osmolarity, and oxygen tension) can be screened for by growing the host under such conditions and supplying a collection of mutated versions of the desired gene on a plasmid. Reductase enzymes with specificity to medium side-chain 3-ketoacyl-CoA's, such as 3-ketohexanoyl-CoA, can be screened for by identifying PHA synthesizing colonies in a strain that has a msc-PHA polymerase gene integrated on the chromosome and mutagenized versions of a *phbB* gene on a plasmid. The combination of different specificity PHA enzymes allows for the screening of a multitude of new substrate specificities. Further permutations of growth conditions allows for screening of enzymes active under sub-optimal conditions or enzymes that are less inhibited by cellular cofactors, such as Coenzyme A and CoA-derivatives, reduced or oxidised nicotinamide adenine dinucleotide or nicotinamide adenine dinucleotide phosphate (NAD, NADP, NADH, and NADPH).

Using the techniques described herein, *E. coli* strains expressing the genes encoding enzymes for the medium side-chain PHA pathway can be constructed. Strains in which either *phaC* or *phaG* or both are integrated on the chromosome *of E. coli* accumulate a PHA including medium chain-length 3-hydroxy fatty acids of which 3-hydroxydecanoate is the predominant constituent. When *phaC* is integrated by itself, msc-PHAs can be synthesized from fatty acids. In such strains, it is advantageous to manipulate fatty acid oxidation such that 3-hydroxy fatty acid precursors accumulate intracellularly. This manipulation can be achieved by mutagenesis or by substituting the *E. coli* fatty acid degradation enzymes FadA and FadB encoding genes with the corresponding *faoAB* genes from *Pseudomonas putida* or related rRNA homogy group I fluorescent pseudomonad.

**Table 1: Phenotypes of Strains for Screening of New or Improved Enzymes**

| **Genes integrated on chromosome** | **Gene(s) on plasmid** | **Carbon source for screen** | **Screen identifies genes encoding** |
|---|---|---|---|
| phbC | library | glucose | new thiolase/ reductase |
| | library | fatty acids | new reductase, hydratase, transferase |
| | library | hydroxy fatty acid, e.g. 4-hydroxybutyrate (4HB) | hydroxy fatty acid activating enzyme, e.g. 4HB-CoA transferase (acetyl-CoA or succinyl-CoA dependent) or 4HB-CoA synthase |
| | phaG | glucose | transferase with new substrate specificity |
| phbAB | library | glucose | new polymerase gene |
| | phaC | glucose; altered environmental conditions | polymerase with new substrate specificity; increased activity under sub-optimal conditions |
| phbBC | library | glucose | new thiolase |
| | phbA | limiting glucose/less prefered carbon sources or rich medium; altered environmental conditions | deregulated thiolase; increased activity under sub-optimal conditions |
| phbAC | library | glucose | new reductase |
| | phbB | limiting glucose/less prefered carbon sources or rich medium; altered environmental conditions | deregulated reductase; increased activity under sub-optimal conditions |
| phbCAB | library | any | enzymes affecting PHB formation under specific conditions |
| phbCAB, random mutations (chemical or transposon) | | any | enzymes affecting PHB formation under specific conditions |
| phaC | library | hexanoate | hydratase with specificity for C6 and longer substrates |
| | phaJ | fatty acids | hydratase with increased specificity for C6 and longer substrates |
| | phbB | fatty acids | reductase with new substrate specificity |
| phaC fadR⁺, Δato | phbAB | glucose + butyrate | thiolase/reductase combination specific for C6 monomer |
| phaJ | phaC | fatty acids | polymerase with wider substrate specificity |
| phaG | phbC | glucose | polymerase with wilder substrate specificity |

### Examples

The methods and compositions described herein will be further understood by reference to the following non-limiting examples. These examples use the following general methods and materials.

### Materials and Methods

*E. coli* strains were grown in Luria-Bertani medium (Sambrook, et al., Molecular Cloning: A Laboratory Manual, 2d ed. (Cold Spring Harbor Laboratory Press, Cold Spring Harbor, NY [1992 at 37°C or 30°C or in minimal E2 medium (Lageveen et al., Appl. Environ. Microbiol. 54: 2924-2932 (1988)). DNA manipulations were performed on plasmid and chromosomal DNA purified with the Qiagen plasmid preparation or Qiagen chromosomal DNA preparation kits according to manufacturers recommendations. DNA was digested using restriction enzymes (New England Biolabs, Beverly, MA) according to manufacturers recommendations. DNA fragments were isolated from 0.7% agarose-Tris/acetate/EDTA gels using a Qiagen kit.

Plasmid DNA was introduced into *E. coli* cells by transformation or electroporation (Sambrook, et al., Molecular Cloning: A Laboratory Manual, 2d ed. (Cold Spring Harbor Laboratory Press, Cold Spring Harbor, NY)). Transposition of *phb* genes from the pUT vectors was achieved by mating of the plasmid donor strain and the recipient (Herrero et al., J. Bacteriol. 172:6557 (1990)). The recipient strains used were spontaneous naladixic acid or rifampicin resistant mutants of *E*. *coli* derived from either LS5218 or MBX23. MBX23 is LJ14 *rpoS::*Tn10 in which the *rpoS*::Tn10 allele was introduced by P1 transduction from strain 1106 (Eisenstark). Recipients in which *phb* genes have been integrated into the chromosome were selected on naladixic acid or rifampicin plates supplemented with the antibiotic resistance specified by the mini-transposon, kanamycin ,or chloramphenicol. Oligonucleotides were purchased from Biosynthesis or Genesys. DNA sequences were determined by automated sequencing using a Perkin-Elmer ABI 373A sequencing machine. DNA was amplified using the polymerase-chain-reaction in 50 microliter volume using PCR-mix from Gibco-BRL (Gaithersburg, MD) and an Ericomp DNA amplifying machine.

DNA fragments were separated on 0.7% agarose/TAE gels. Southern blots were performed according to procedures described by Sambrook, et al., Molecular Cloning: A Laboratory Manual, 2d ed. (Cold Spring Harbor Laboratory Press, Cold Spring Harbor, NY). Detection of DNA fragments containing *phb* genes was performed using chemiluminescent labeling and detection kits from USB/Amersham. Proteins samples were denatured by incubation in a boiling water bath for 3 minutes in the presence of 2-mercaptoethanol and sodium dodecylsulphate and subsequently separated on 10%, 15%, or 10-20% sodium dodecylsulphate-polyacrylamide gels. After transfer of protein to supported nitrocellulose membranes (Gibco-BRL, Gaithersburg, MD), 3-ketoacyl-CoA thiolase, acetoacetyl-CoA reductase and PHB polymerase was detected using polyclonal antibodies raised against these enzymes and horseradish peroxidase labeled secondary antibodies followed by chemiluminescent detection (USB/Amersham).

Acetoacetyl-CoA thiolase and acetoacetyl-CoA reductase activities were determined as described by Peoples and Sinskey, J. Biol. Chem. 264: 15293-15297 (1989) in cell free extracts from strains grown for 16 hours in LB-medium at 37 C. The acetoacetyl-CoA thiolase activity is measured as degradation of a Mg²⁺-acetoacetyl-CoA complex by monitoring the decrease in absorbance at 304 nm after addition of cell-free extract using a Hewlett-Packer spectrophotometer. The acetoacetyl-CoA reductase activity is measured by monitoring the conversion of NADH to NAD at 340 nm using a Hewlett-Packer spectrophotometer.

Accumulated PHA was determined by gas chromatographic (GC) analysis as follows. About 20 mg of lyophilized cell mass was subjected to simultaneous extraction and butanolysis at 110 C for 3 hours in 2 mL of a mixture containing, by volume, 90% 1-butanol and 10% concentrated hydrochloric acid, with 2 mg/mL benzoic acid added as an internal standard. The water-soluble components of the resulting mixture were removed by extraction with 3 mL water. The organic phase (1 µL at a split ratio of 1:50 at an overall flow rate of 2 mL/min) was analyzed on an HP 5890 GC with FID detector (Hewlett-Packard Co, Palo Alto, CA) using an SPB-1 fused silica capillary GC column (30 m; 0.32 mm ID; 0.25 µm film; Supelco; Bellefonte, PA) with the following temperature profile: 80°C, 2 min.; 10°C per min. to 250°C; 250°C, 2 min. The standard used to test for the presence of 4-hydroxybutyrate units in the polymers was γ-butyrolactone, which, like poly(4-hydroxybutyrate), forms n-butyl 4-hydroxybutyrate upon butanolysis. The standard used to test for 3-hydroxybutyrate units in the polymer was purified PHB.

The molecular weights of the polymers were determined following chloroform extraction by gel permeation chromatography (GPC) using a Waters Styragel HT6E column (Millipore Corp., Waters Chromatography Division, Milford, MA) calibrated versus polystyrene samples of narrow polydispersity. Samples were dissolved in chloroform at 1 mg/mL, and 50 µL samples were injected and eluted at 1 mL/min. Detection was performed using a differential refractometer.

1-Methyl-3-nitro-1-nitroso-guanidine (NTG) mutagenesis was performed as described by Miller, A Short Course in Bacterial Genetics (Cold Spring Harbor Laboratory Press, Cold Spring Harbor, NY) using a 90 minute treatment with 1 mg/ml NTG corresponding to 99% killing.

**Example 1: Host Strains and Plasmid Tools for Gene Integration.**

Strains and plasmids from which transposon vectors and transposon derivatives were developed are listed in Tables 2 and 3 below. MBX245 and MBX247 were selected by growing MBX23 and LS5218 respectively on LB plates containing approximately 30 g/ml naladixic acid. MBX246 and MBX248 were selected by growing MBX23 and LS5218, respectively, on LB plates containing 50 g/ml rifampicin. Colonies that appeared on these selective media within 24 hours were replica plated on the same medium and after growth stored in 15% glycerol/nutrient broth at -80°C.

MBX245 and MBX247 were selected by growing MBX23 and LS5218 respectively on LB plates containing 30 µg/ml nalidixic acid. MBX246 and MBX248 were selected by growing MBX23 and LS5218 respectively on LB plates containing 50 µg/ml rifampicin. Colonies that appeared on these selective media within 24 hours were replica plated on the same medium and after growth stored in 15% glycerol/nutrient broth at -80 °C.

**Table 2: Host Strains Used For Gene Integration**

| **strain** | **genotype** | **source** |
|---|---|---|
| DH5α | recA1 endA1 gyrA96 thi hsdR17 supE44 relA1 Δ(lac-proAB) (Φ80dlaΔ(lacZ)M15) | 1 |
| S17-1λpir | recA thi pro hsdR⁻M⁺ RP4:2-Tc::Mu::Km Tn7 λpir lysogen | 2 |
| CC118 λpir | Δ(ara-leu) araD ΔlacX74 galE galK phoA20 thi-1 rpsE rpoB argE(Am) recA1, λ pir lysogen | 2 |
| XLl-Blue | F'::Tn10 lacI^{q} Δ(lacZ) M15 proAB/recA1 endA1 gyrA96 thi hsdR17 supE44 relA1 Δ(lac-proAB) | 3 |
| LS5218 | fadR601 atoC512^{c} | Spratt et al, 1981 J. Bacteriol. 146: 1166-1169 |
| LJ14 | LS5218 atoC2^{c} atoA14 | Spratt et al, 1981 J. Bacteriol. 146: 1166-1169 |
| MBX23 | LJ14 rpoS | Metabolix, Inc. |
| MBX245 | MBX23 NI^{r} | Metabolix, Inc. |
| MBX246 | MBX23 Rf | Metabolix, Inc. |
| MBX247 | LS5218 NI^{r} | Metabolix, Inc. |
| MBX248 | LS5218Rf^{r} | Metabolix, Inc. |

| | | |
|---|---|---|
| 1. New England Biolabs (Beverly, MA) 2. Herrero et al., J. Bacteriol. 172:6557-67 (1990) 3. Stratagene (San Diego, CA) | | |

**Table 3: Plasmids Used For Gene Integration**

| **plasmid** | **characteristics** | **source** |
|---|---|---|
| pUC18Not | Ap^{r}, NotI sites flanking polylinker | 2 |
| pUC18Sfi | Ap^{r}, SfiI sites flanking polylinker | 2 |
| pUTkan | Ap^{r}, Km^{r}, oriR6K, mobRP4 depends on λpir for replication | 2 |
| pUTHg | Ap^{r}, Hg^{r}, oriR6K, mobRP4 depends on λpir for replication | 2 |
| pKPS4 | Ap^{r}, *phaCl* from *Pseudomonas oleovorans* | |
| pUCDBK1 | Ap^{r}, *phbA* and *phbB* from *Zoogloea ramigera* | Peoples and Sinskey 1989, Molecular Microbiol.3: 349-357 |
| pZS | Ap^{r}, *phbC* from *Zoogloea ramigera* | WO 99/14313 |

### Example 2: Construction of Cloning Vectors to Facilitate Integration of phb Genes.

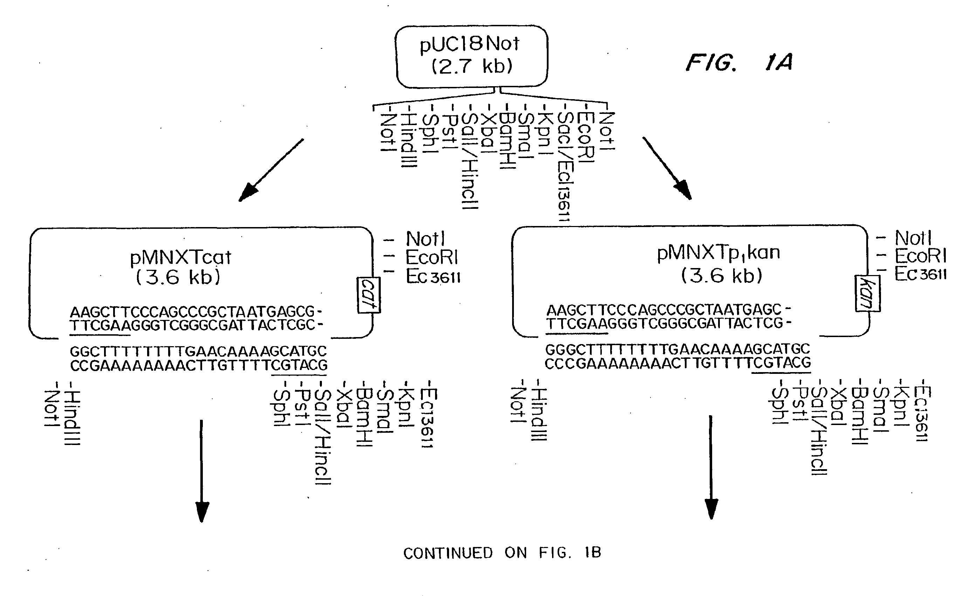

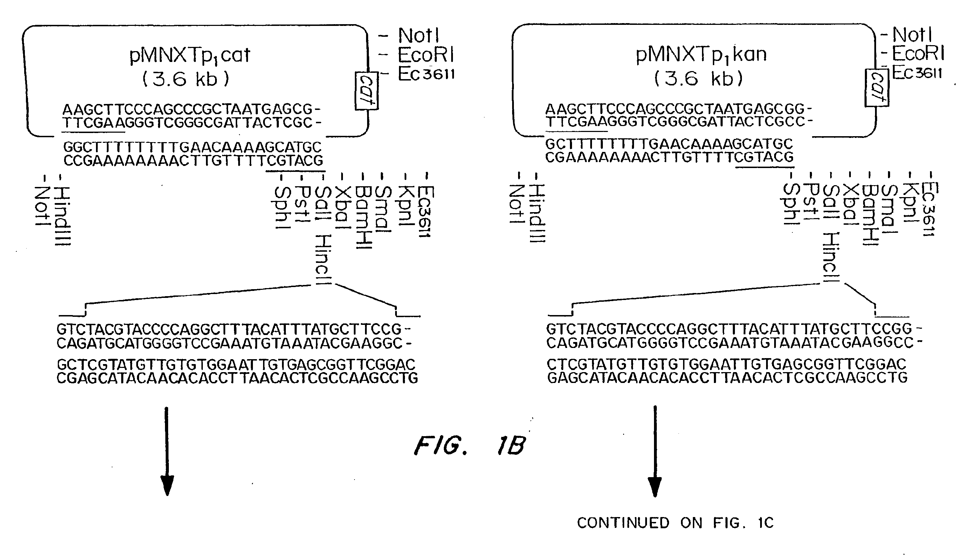

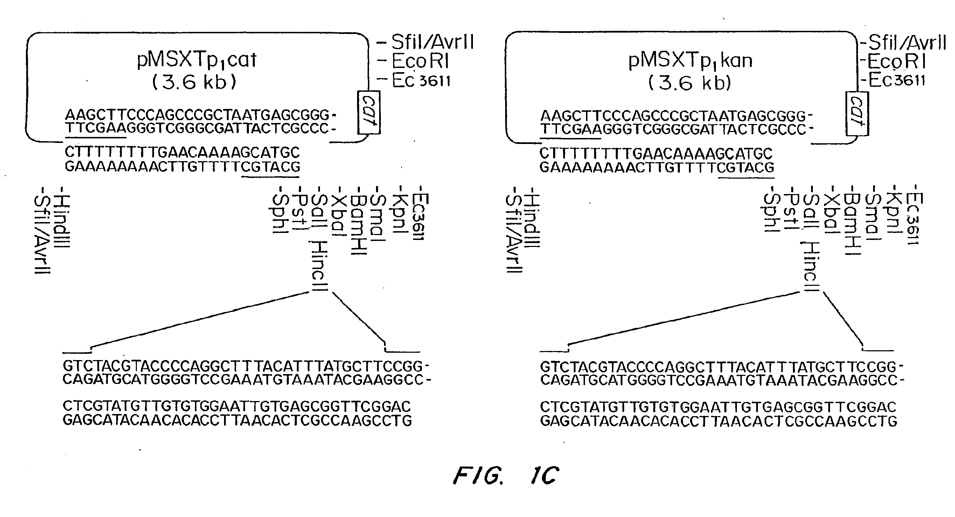

The plasmids pMNXTp1kan and pMTXTp1cat were based on the plasmids pUC18Not and pUC1BSfi and developed as shown in Figure 1.

The Tn903 kanamycin (Km) resistance gene from plasmid PUGS18 was amplified by PCR using the oligonucleotide primers linkK1, 5' TGCATGCGATATCAATTGTCCA GCCAGAAAGTGAGG, and linkK2, 5' ATTTATTCAACAAAGCCGCC.

Prior to PCR amplification, the primers were phosphorylated using T4 polynucleotide kinase using standard procedures. The DNa was amplified using the following program: 1 cycle of 3 min at 95 °C, 40 s at 42 °C, 2 min at 72 °C, followed by 30 cycles of 40 s at 95 °C, 40 s at 42 °C and 90 s at 72 °C. The DNA then was phenol extracted and treated with T4 DNA polymerase prior to gel purification. The blunt ended 0.8 kb DNA fragment was then inserted into the *Ecl136II* site in the polylinker of pUC18Not to obtain pMNXkan.

The *cat* gene was obtained as an *HindIII* cassette from Pharmacia (Pharmacia Inc. NJ), blunt ended using Klenow fragment of DNA polymerase, and inserted into the *Ecl136II* site of pUC18Not to obtain pMNXcat.

The *trp* terminator sequence was constructed by annealing the two synthetic oligonucleotides TERM1 (5' CCCAGCCCGCTAATGAGCGGGCTTTTTTTTGAACAAAA 3') and TERM2 (5' TACGTATTTTGTTCAAAAAAAAGCCCGCTCATTAGCGGG CTGGG 3').

The terminator was then inserted into the *HindIII-SphI* site of pMNXkan and pMNXcat to obtain pMNTkan and pMNXTcat, respectively. These vectors were constructed such that any promoter fragment can be added between the *SphI* and *SacI* sites. Promoter p₁ was constructed by annealing of the synthetic oligonucleotides PHBB1 (5' TACGTACCCCAGGCTTTACATTTATGCTTCCGGCTCGTATGTTGT GTGGAATTG TGAGCGGTT 3') and PHBB2 (5'TTCGAACCGCTCACAATTCCACACAACATACGAGCCGGAAGC ATAAATGTAAAGCCTGGGG 3') followed by filling in the ends with Klenow fragment of DNA polymerase. The blunt-ended promoter fragment p₁ was then inserted into the *HincII* site of pMNXTkan and pMNXTcat to obtain pMNXTp₁kan and pMNXTp₁cat, respectively.

Plasmid pMSXTp₁cat was constructed by transferring the Tp₁cat cassette from pMNXTp₁cat as an *EcoRI-HindIII* fragment into the *EcoRI-HindIII* site of pUC18Sfi. Similarly, pMSXTp₁kan was constructed by transferring the *EcoRI-HindIII* fragment containing Tp₁kan into the *EcoRI-HindIII* site of pUC18Sfi.

### Example 3: Construction of Plasmids for Chromosomal Integration of phbC, encoding PHB polymerase.

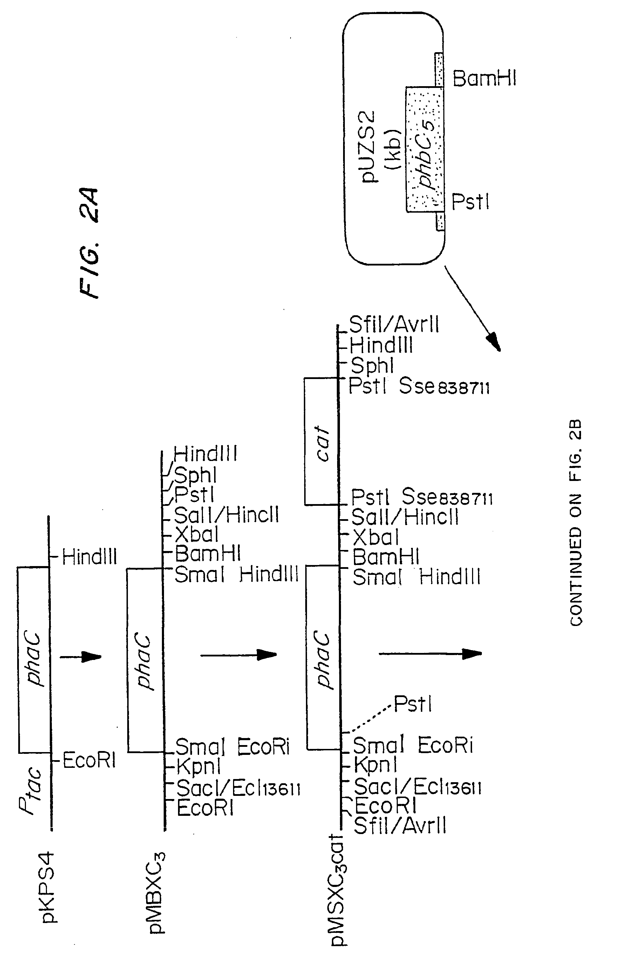

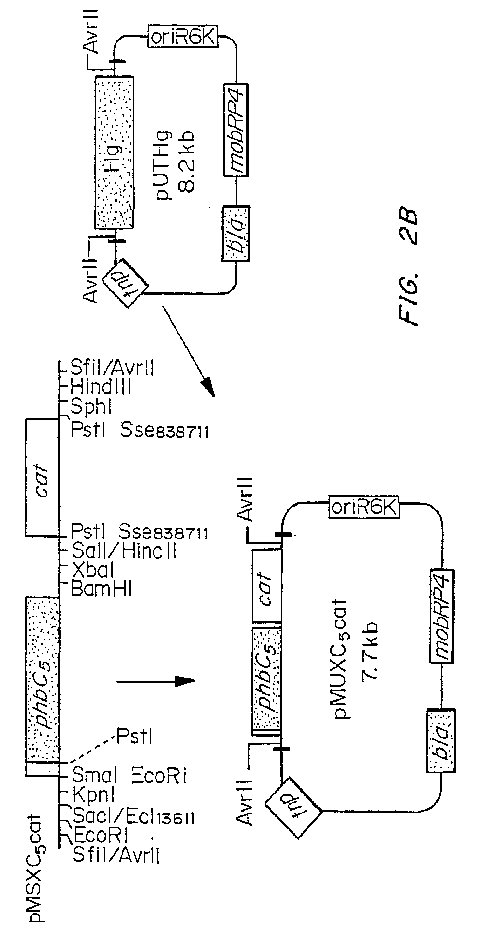

Plasmid pMUXC₅cat contains the *phbC* gene from *Z*. *ramigera* on a transposable element for integration of this gene on the chromosome of a recipient strain, as shown in Figure 2. Strong translational sequences were obtained from pKPS4 which includes *phaCl* encoding PHA polymerase from *P*. *oleovorans* in the pTrc vector (Pharmacia). In this construct, *phaCl* is preceded by a strong ribosome binding site: AGGAGGTTTTT(-ATG). The *phaCI* gene including the upstream sequences, was cloned as a blunt ended *EcoRI-HindIII* fragment in the *SmaI* site of pUC18Sfi to give pMSXC₂. A blunt ended *cat* gene cassette was subsequently cloned in the blunt-ended *Sse8387II* site, resulting in pMSXC₃cat. At this point, all of the *phaC1* coding region except the 5' 27 base pairs were removed as a *PstI-BamHI* fragment and replaced by the corresponding fragment from the *phbC* gene from Z. *ramigera.* The resulting plasmid pMSXC₅cat encodes a hybrid PHB polymerase enzyme with the 9 amino terminal residues derived from the *P. oleovorans* PHA polymerase and the remainder from Z. *ramigera*. The C₅cat cassette was then excised as an *AvrII* fragment and cloned in the corresponding sites of pUTHg, thereby deleting the mercury resistance marker from this vector. The resulting plasmid, pMUXC₅cat, contains a C₅cat mini-transposon in which *phbC* is not preceded by a promoter sequence. Expression of the cassette upon integration is therefore dependent on transcriptional sequences that are provided by the DNA adjacent to the integration site.

### Example 4: Construction of Plasmids for Chromosomal Integration of phbAB, encoding thiolase and reductase.

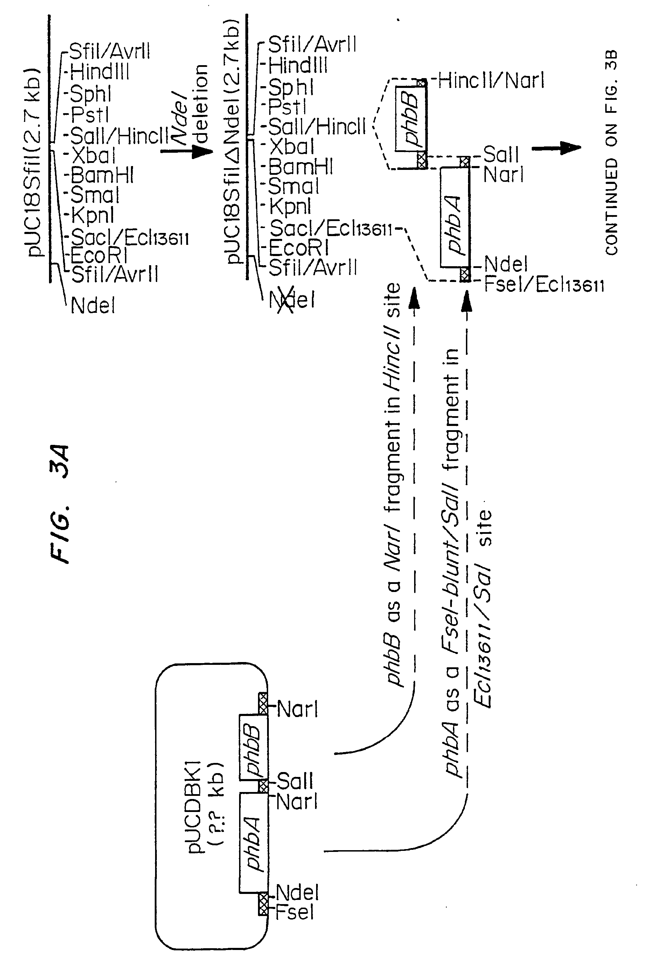

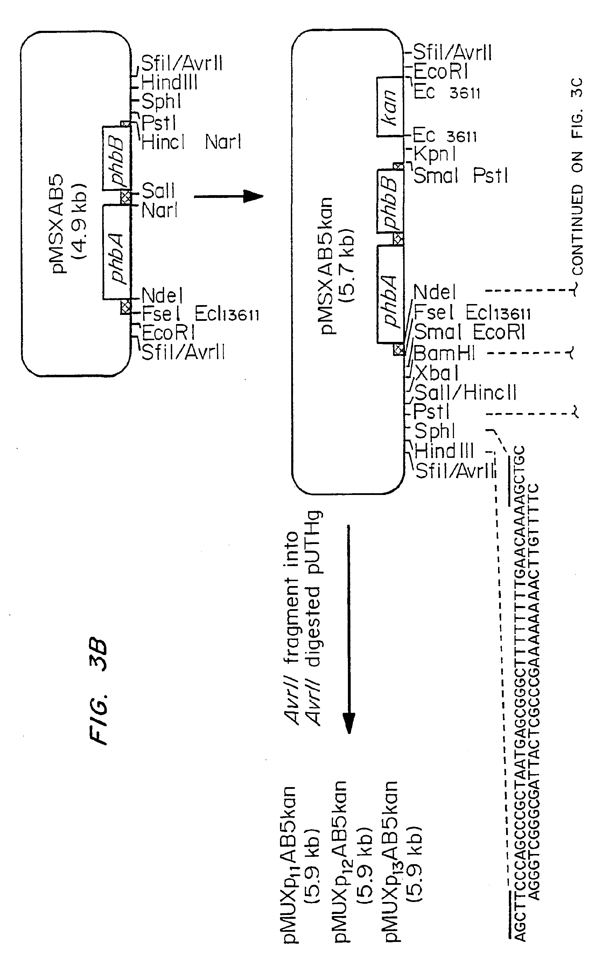

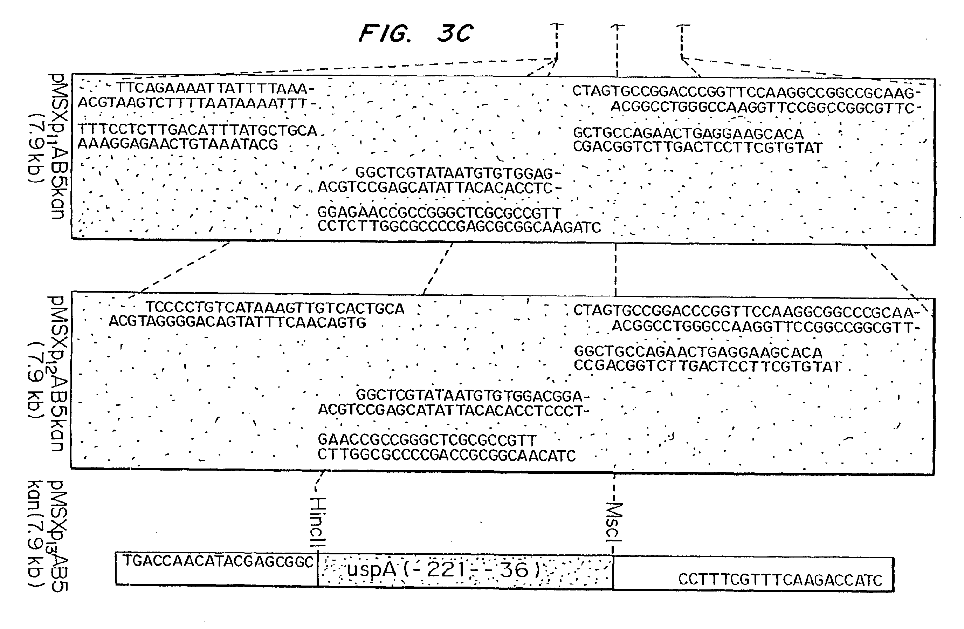

pMSXTp₁AB₅kan2 was constructed from pMSXTp₁kan as partially shown in Figure 3. First pMSXTp₁kan was digested with *NdeI,* filled in with Klenow and religated to obtain pMSXTp₁kan2 in which the *NdeI* site is deleted. This deletion results in a unique *NdeI* site just upstream of *phbA* of *Z. ramigera* during later stages of the cloning procedure.

B₅ was cloned as a *NarI* fragment from pUCDBK1 (Peoples and Sinskey 1989, Molecular Microbiol. 3: 349-357) and cloned in the *HincII* site of pUC18Sfi to generate pMSXB₅. A₅ was inserted as an *FseI*/blunt-*SalI* fragment in the *Ecl136II-SalI* sites resulting in pMSXAB₅ and regenerating the *Z*. *ramigera* AB₅ intergenic region. pMSXAB₅cat was created by inserting a promoterless *cat* cassette in the *HindIII site* of pMSXAB₅. The AB₅ fragment from pMSXAB₅cat was cloned as a *EcoRI-PstI* fragment into the *SmaI* site of pMSXTp₁kan2 giving pMSXTp₁AB₅kan2.

The expression cassette AB₅cat was then excised as a 2.8 kb *AvrII* fragment and ligated into the *AvrII* site of pUTHg and transformed into *E. coli* strain CC118 λpir to obtain plasmid pMUXAB₅cat. This plasmid was then transformed into *E. coli* S17-1λpir and used to insert the AB5cat expression cassette into the chromosome *of E. coli* MBX247 by conjugation. The resulting Ap^{s}/ Cm^{r} transconjugants were characterized for integration and expression of the thiolase and reductase genes encoded by the *phbAB* genes.

### Example 5: Construction of Plasmids with Improved Promoters for Integration of phbAB into the Chromosome of E. coli.

Expression of *phbAB5* was improved by introduction of strong promoters upstream of these genes, as shown in Figure 3. These promoters were generated with sets of oligonucleotides that provide upstream activating sequences, a -35 promoter region, a -10 promoter region with transcriptional start site(s), and mRNA sequences with possible stabilizing functions. Plasmid pMSXTp₁AB₅kan2 was digested with *PstI*/*XbaI,* and a fragment containing the -10 region of the *lac* promoter was inserted as a fragment obtained after annealing oligonucleotides 3A (5' GGCTCGTATAATGTGTGGAGGGAGAACCGCCGGGCTCGCGCCGTT) and 3B (5' CTAGAACGGCGCGAGCCCGGCGGTTCTCCCTCCACACATTATAC GAGCCTGCA).
Next, a fragment containing the *lac* -35 region and the *rrnB* region were inserted into the *PstI* site as a fragment obtained after annealing the oligonucleotides:
1A (5' TTCAGAAAATTATTTTAAATTTCCTCTTGACATTTATGCT GCA) and 1B (5'GCATAAATGTCAAGAGGAAATTTAAAATAATTTTCTGAATGCA).
Next, the messenger stabilizing sequence including the transcriptional start site from AB₅ was inserted into the *XbaI-NdeI* sites as a fragment obtained after annealing the oligonucleotides:
4A (5'CTAGTGCCGGACCCGGTTCCAAGGCCGGCCGCAAGGCTGCCAG AACTGAGGAAGCACA) and 4B (5'TATGTGCTTCCTCAGTTCTGGCAGCCTTGCGGCCGGCCTTGGAA CCGGGTCCGGCA).
The resulting plasmid is pMSXp₁₁AB₅kan2. The *AvrII* fragment, containing Tp₁₁AB₅kan2 was cloned into pUTHg cut with *AvrII* and used for integration into the genome of MBX379 and MBX245.

Plasmid pMSXTp₁₂AB₅kan2 was constructed as pMSXTP₁₁AB₅kan2 with the distinction that the following oligonucleotides were used instead of oligonucleotides 1A and 1B: 2A:
(5' TCCCCTGTCATAAAGTTGTCACTGCA)
   and 2B
(5' GTGACAACTTTATGACAG GGGATGCA).
These oligonucleotides provide a consensus *E. coli pho* box and -35 promoter region to generate a promoter that is potentially regulated by the phosphate concentration in the medium.

pMSXTp₁₃AB₅kan2 was constructed to provide expression of AB₅ from a promoter that has been shown to be expressed under general stress conditions such as nutrient limitation, pH or heat shock, and administration of toxic chemicals. The promoter region of *uspA* was amplified using oligonucleotides UspUp
(5' TGACCAACATACGA GCGGC)
and UspDwn
(5' CTACCAGAACTTTGCTTTCC)
in a PCR reaction consisting of an incubation at 95 C for 3 min. followed by 30 cycles of 40 s at 95 °C, 40 s at 42 °C, an incubation for 7 min. at 68 °C, and final storage at 4 °C. The approximately 350 bp PCR product was cloned into pCR2.1 (Invitrogen Corp., USA) to generate pMBXp₁₃. An approximately 190 bp *HincII-MscI* fragment containing the promoter and transcriptional start site for *uspA* and the first 93 bp of the *uspA* mRNA was cloned into blunt ended *BamHI-Sse8387I* pMSXTp₁kan2 to give pMSXTp₁₃kan2. Plasmid pMSXTp₁₃kan2 was then *KpnI* digested, blunt ended with T4 polymerase and dephosphorylated using calf intestinal phosphatase. The AB₅ genes were isolated as a 2.0 kb *EcoRI*/*Sse8387I* fragment from pMSXAB₅, blunt ended using Klenow and T4 polymerase and ligated into the *KpnI* site of pMSXTp₁₃kan2. In the resulting plasmid pMSXTp₁₃AB₅kan2, the *phbAB* and *kan* genes are expressed from the *uspA* (p₁₃)promoter.

The pₙAB₅kan (n = 11, 12, 13) expression cassettes were then excised as 2.8 kb *AvrII* fragments and ligated into the *AvrII* site ofpUTHg and transformed into *E. coli* strain CC118 λpir to obtain plasmid pMUXpₙAB₅kan. This plasmid was then transformed into *E. coli* S17-1λpir and used to insert p₁₁AB₅kan, p₁₂AB₅kan, and p₁₃AB₅kan expression cassettes into the chromosome of *E. coli* strains by conjugation.

### Example 6: Integration of C₅cat into the Chromosome of E. coli.

C₅cat was introduced into the chromosome of MBX23 by conjugation using S17-1 λpir (pMUXC₅cat) as the donor strain. The conjugation mixture was spread on LB/Nl/Cm plates and integrants were obtained, 40% of which were sensitive to ampicillin, indicating that no plasmid was present in these strains. Five integrants were transformed with pMSXAB₅cat (Ap^{r}) and grown on LB/Ap/Cm/2% glucose to examine biosynthetic activity of PHB polymerase (Table 4).

**Table 4: Integrated Strains**

| **strain** | **strain containing pMSXAB5cat** | **PHB phenotype** | **strain after plasmid curing** |
|---|---|---|---|
| MBX300 | MBX305 | ++++ | MBX325 |
| MBX301 | MBX308 | +++ | MBX331 |
| MBX302 | MBX310 | ++++ | MBX326 |
| MBX303 | MBX315 | ++++ | MBX327 |
| MBX304 | MBX316 | + | MBX337 |

### Example 7: Amplification of C5 Expression in Integrated Strains.

Expression of PHB polymerase was increased by restreaking MBX326 successively on LB plates containing 100, 200, 500, and 1000 µg/ml chloroamphenicol. Strain MBX379 was derived from MBX326 and exhibited chloramphenicol resistance up to 1000 µg/ml. In Southern blot analysis of chromosomal DNA isolated from MBX379 and its predecessors, the *phbC5* copy-number had not increased. Western blot analysis indicated a strong increase in PHB polymerase levels in cell free extracts of these strains when the *phbAB* genes were present on a plasmid.

### Example 8: Integration of p₁₁AB₅kan, p₁₂AB₅kan and p₁₃AB₅kan into MBX379.

S17-1 λpir strains with either pMUXp₁₁AB₅kan, pMUXₚ₁₂AB₅kan, or pMUXp₁₃AB₅kan were mated with MBX379. Transgenic strains in which *phbAB₅kan* had integrated on the chromosome were selected on LB/Nl/Km plates. Among the integrants, PHB producers were identified on LB/glucose plates. Representatives of the individual constructs were MBX612 (MBX379::p₁₁AB₅kan), MBX677 (MBX379:: p₁₂AB₅kan), and MBX680 (MBX379::p₁₃AB₅kan). Southern blots and Western blots showed that the *phbAB* genes had integrated in the chromosome and were expressed in these strains as well. Table 5 shows the PHB accumulation levels of transgenic *E. coli* PHB producers grown in Luria-Bertani medium with 2% glucose or minimal E2 medium with 2% glucose and 0.5% corn steep liquor.

**Table 5: PHB Accumulation Levels for Transgenic E. coli PHB Producers**

| **strain** | **%PHB of cell dry weight** | |
|---|---|---|
| | **LB/glucose** | **E2 glucose** |
| MBX612 | 56 | 35 |
| MBX677 | 58 | 38 |
| MBX680 | 39 | 50 |

### Example 9: Selection and Bacteriophage P1 Transduction to yield Improved Strains.

The growth characteristics of MBX612, 677, and 680 were improved by bacteriophage P1 transduction. A single transduction step was required to transduce the C₅cat and AB₅kan alleles from the different strains into LS5218, indicating that the two separate integration cassettes were located close to each other on the chromosome. The resulting strains are MBX690 (from MBX681), MBX691 (from MBX677), and MBX698 (from MBX680). Repeated inoculation of MBX612 on minimal E2 medium with limiting nitrogen resulted in MBX681. Unlike the strains generated by P1 transduction, MBX681 did not exhibit improved growth characteristics. Southern blots and Western blots show that *phbC* and the *phbAB* genes were successfully transduced and were expressed in these strains as well. Table 6 below shows PHB accumulation levels for these transgenic *E. coli* PHB producers grown in Luria-Bertani medium with 2% glucose or minimal E2 medium with 2% glucose and 0.5% corn steep liquor.

**Table 6: PHB Accumulation Levels for Transgenic E. coli PHB Producers**

| **strain** | **%PHB of cell dry weight** | |
|---|---|---|
| | **LB/glucose** | **E2 glucose** |
| MBX681 | 54 | 22 |
| MBX690 | 52 | 44 |
| MBX691 | 54 | 28 |
| MBX698 | 37 | 15 |

### Example 10: Further Improvements of Transgenic E. coli Strains for PHB production

Mutagenesis using NTG or EMS was used to further improve PHB production in MBX680. Strains MBX769 and MBX777 were selected after treatment of MBX680 with EMS and NTG, respectively. These strains were found to be able to grow on R2-medium supplied with 1% glucose, 0.5% corn steep liquor, and 1 mg/ml chloroamphenicol. MBX769 was grown in 50 ml R-10 medium/0.5% CSL with 2 or 3% glucose at 37 °C for 20 to 26 hours. PHB was accumulated to 71% of the cell dry weight. Similarly, MBX769 was grown in 50 ml LB with or without 0.375 g/L KH₂PO₄, 0.875 K₂HPO₄, 0.25 (NH₄)₂SO₄, and a total of 50 g/L glucose (five aliquots were added over the course of the incubation). After 63 hours of incubation, PHB had accumulated up to 96% of the cell dry weight.

The *phbC* and *phbAB* alleles from MBX777 were subsequently transduced into LS5218, resulting in MBX820. Southern blots and Western blots show that *phbC* and the *phbAB* genes were successfully transduced and were expressed in these strains as well. Table 7 shows the PHB accumulation levels of these transgenic *E*. *coli* PHB producers grown in Luria-Bertani medium with 2% glucose or minimal E2 medium with 2% glucose and 0.5% corn steep liquor.

**Table 7: PHB Accumulation Levels for Transgenic E. coli PHB Producers**

| **strain** | **%PHB of cell dry weight** | |
|---|---|---|
| | **LB/glucose** | **E2 glucose** |
| MBX680 | 39 | 50 |
| MBX777 | 67 | 57 |
| MBX820 | 53 | 50 |

### Example 11: Growth Characteristics of Transgenic E. coli PHB Producers.

The introduction of *phb* genes into MBX245 (t_{d} = 47 min.) was accompanied by a reduction in growth rate (MBX680, t_{d} = 71 min.). Improved PHB production was achieved by EMS mutagenesis, but did not improve the growth rate (MBX777, t_{d} = 72 min.). P1 transduction of the PHB genes into a wild-type strain (MBX184) resulted in the same high growth rate as exhibited by MBX245 and PHB accumulation up to 50% of the cell dry weight in less than 24 hours (MBX820, t_{d} = 45 min.).

### Example 12: Plasmids for Chromosomal Integration of Other pha Genes.

The integration of *phbC, phbA,* and *phbB* from Z. *ramigera* described herein also is applicable to other *pha* genes, such as genes encoding PHB polymerase from *R. eutropha* (C1), PHA polymerase from *P. oleovorans* (C3), PHB polymerase from *A. caviae* (C12), ACP::CoA transacylase from *P. putida* (G3), (R)-specific enouyl-CoA hydratase from *A*. *caviae* (J12), a broad substrate specific 3-ketoacyl-CoA thiolase from *R. eutropha* (A1-II), or a phasin from *R. eutropha* (P1-I and P1-II). These genes were obtained by polymerase chain reaction amplification using the following primers:
C1 up 5' g-GAATTC-aggaggtttt-ATGGCGACCGGCAAAGGCGCGGCAG 3'
C1 dw 5' GC-TCTAGA-AGCTT-tcatgccttggctttgacgtatcgc 3'
C3 up 5' g-GAATTC-aggaggtttt-ATGAGTAACAAGAACAACGATGAGC 3'
C3 dw 5' GC-TCTAGA-AGCTT-tcaacgctcgtgaacgtaggtgccc 3'.
C12 up 5' g-GAATTC-aggaggtttt-ATGAGCCAACCATCTTATGGCCCGC 3'
C12 dw 5' GC-TCTAGA-AGCTT-TCATGCGGCGTCCTCCTCTGTTGGG3'
G3 up 5' g-GAATTC-aggaggtttt-ATGAGGCCAGAAATCGCTGTACTTG 3'
G3 dw 5' GC-TCTAGA-AGCTT-tcagatggcaaatgcatgctgcccc 3'
J12 up 5' ag-GAGCTC-aggaggtttt-ATGAGCGCACAATCCCTGGAAGTAG3'
J12 dw 5' GC-TCTAGA-AGCTT-ttaaggcagcttgaccacggcttcc 3'
A1-II up 5' g-GAATTC-aggaggtttt-ATGACGCGTGAAGTGGTAGTGGTAAG 3'
A1-II dw 5' GC-TCTAGA-AGCTT-tcagatacgctcgaagatggcggc 3'.
P1-I up 5' g-GAATTC-aggaggtttt-ATGATCCTCACCCCGGAACAAGTTG 3'
P1-I dw 5' GC-TCTAGA-AGCTT-tcagggcactaccttcatcgttggc 3'
P1-II up 5' g-GAATTC-aggaggtttt-ATGATCCTCACCCCGGAACAAGTTG 3'
P1-II dw 5' GC-TCTAGA-AGCTT-tcaggcagccgtcgtcttctttgcc 3'
PCR reactions included 10 pmol of each primer, 1 to 5 µl of chromosomal DNA or boiled cells, and 45 µl PCR mix from Gibco BRL (Gaithersburg, MD). Amplification was by 30 cycles of 60 s incubation at 94 C, 60 s incubation at a temperature between 45 C and 68 C and 1 to 3 minutes incubation at 72 C. PCR products were purified, digested with EcoRI and HindIII, blunt ended with the Klenow fragment of DNA polymerase, and cloned in the SmaI site of pMSXcat, pMSXkan, pMNXcat, or pMNXkan according to the schemes shown in Figures 1 and 2. pMUX*pha* was derived from pUTHg or pUTkan; and pMLX*pha* was derived from pLOFHg, where *pha* stands for the *pha* gene of choice. These plasmids were used for integration of the desired *pha* gene into the chromosome of *E*. *coli* or any other Gram-negative microbial strain suitable for PHA production

### Example 13: PHBV Copolymer Producing Transgenic E. coli Strains.

*E. coli* strains with chromosomally integrated *phb* genes such as described above also can be used to produce PHBV copolymers. PHBV is generally synthesized in fermentation systems where propionic acid is co-fed with glucose or other carbohydrate. After uptake, propionate is converted to propionyl-CoA, which by the action of acyl-CoA thiolase and 3-ketoacyl-CoA reductase is converted to 3-hydroxyvaleryl-CoA (3HV-CoA). 3HV-CoA is subsequently polymerized by PHA polymerase.

The capacity to accumulate PHBV can be increased by increasing levels of enzymes that specifically synthesize HV monomers. Such enzymes may be involved in the uptake of propionic acid, in the activation of propionic acid to propionyl-CoA or in any of the PHB biosynthetic enzymes. Additionally, alternative enzymes can be isolated from other sources, or propionyl-CoA can be obtained from alternative pathways, e.g. from the methylmalonyl-CoA pathway. In this pathway, succinyl-CoA is converted to methylmalonyl-CoA which is then decarboxylated to yield propionyl-CoA.

### Example 14: PHB-4HB Copolymer Producing Transgenic E. coli Strains.

Homopolymers and copolymers containing 4HB monomers can be produced by transgenic *E. coli* strains. Incorporation of 4HB from 4HB-CoA can be achieved by feeding 4-hydroxybutyrate to the PHA producing organisms. 4HB is activated to 4HB-CoA either through a 4-hydroxybutyryl-CoA transferase such as *hbcT* (OrfZ) from *Clostridium kluyveri* or by an endogenous *E*. *coli* enzyme or by any other enzyme with this capability. A P4HB homopolymer is produced when the transgenic *E*. *coli* strain contains only the *phbC* gene. 4HB containing copolymers can be synthesized when the transgenic *E. coli* strain contains genes encoding the complete PHB biosynthetic pathway.

*E. coli* MBX821 (LS5218::C₅-cat³⁷⁹, atoC^{c}) was grown in Luria-Bertani medium and resuspended in 100 ml 10% LB with 5 g/L 4HB and 2 g/L glucose. After incubation of this culture for 24 hours, PHA was characterized and identified as containing only 4HB monomers. Similarly, *E. coli* MBX777 with a plasmid containing *hbcT* such as pFS 16, was grown in LB/4HB (5 g/L) and the resuting polymer was identified as PHB4HB with 35.5 % 4HB monomers.

### Example 15: Production of poly(4-hydroxybutyrate) from 4-hydroxybutyrate in recombinant E. coli with no extrachromosomal DNA.

Poly(4-hydroxybutyrate) can be synthesized from 4-hydroxybutyrate by *E. coli* expressing 4-hydroxybutyryl-CoA transferase (*hbcT*) and PHA synthase (*phaC*) genes from a plasmid. If these genes are integrated into the *E. coli* chromosome and expressed at high levels, the recombinant *E. coli* should be able to synthesize poly(4-hydroxybutyrate) from 4-hydroxybutyrate. The *hbcT and phbC* genes were inserted into pUTHg (Herrero, et al., J. Bacteriol. 172:6557-67, 1990) as follows. pMSXC₅cat and pFS16 were both digested with *Bam*HI and *Sal*I*.* The large fragment of pMSXC₅cat and the fragment of pFS16 containing the *hbcT* gene thus obtained were ligated together using T4 DNA ligase to form pMSXC₅hbcT-cat. The fragment containing the *phaC*, *hbcT,* and *cat* genes was removed from pMSXC₅hbcT-cat by digestion with *Avr*II, and it was inserted using T4 DNA ligase into pUTHg that had been digested with *Avr*II and treated with calf intestinal alkaline phosphatase to prevent self-ligation. The plasmid thus obtained was denoted pMUXC₅hbcT-cat. The plasmid pMUXC₅hbcT-cat was replicated in MBX129 and conjugated into MBX1177. The strain MBX1177 is a spontaneous mutant of *E. coli* strain DH5α that was selected for its ability to grow on minimal 4-hydroxybutyrate agar plates. MBX1177 is also naturally resistant to nalidixic acid. The recipient cells were separated from the donor cells by plating on LB-agar supplemented with 25 µg/mL chloramphenicol and 30 µg/mL nalidixic acid. Survivors from this plate were restreaked on minimal medium, containing, per liter: 15 g agar; 2.5 g/L LB powder (Difco; Detroit, Michigan); 5 g glucose; 10 g 4-hydroxybutyrate; 1 mmol MgSO₄; 10 mg thiamine; 0.23 g proline; 25.5 mmol Na₂HPO₄; 33.3 mmol K₂HPO₄; 27.2 mmol KH₂PO₄; 2.78 mg FeSO₄-7H₂O; 1.98 mg MnCl₂·4H₂O; 2.81 mg CoSO₄·7H₂O; 0.17 mg CuCl_{2·}2H₂O; 1.67 mg CaCl₂·2H₂O; 0.29 mg ZnSO₄-7H₂O; and 0.5 mg chloramphenicol. Colonies from this plate that appeared to be especially white and opaque were evaluated in shake flasks containing the same medium as above except without agar. The individual colonies were first grown in 3 mL of LB medium for 8 hours, and 0.5 mL of each culture was used to inoculate 50 mL of the medium described above. These flasks were incubated at 30 °C for 96 hours. One isolate was found by GC analysis (for which the cells were removed from the medium by centrifugation for 10 minutes at 2000 x g, washed once with water and centrifuged again, then lyophilized) to contain 4.9% poly(4-hydroxybutate) by weight. This strain was denoted MBX1462 and selected for further manipulations. MBX1462 was treated with the mutagen 1-methyl-3-nitro-1-nitrosoguanidine (MNNG), a chemical mutagen, by exposing a liquid culture of MBX1462 to 0.1 mg/mL MNNG for 90 minutes. It was found that 99.8% of the cells were killed by this treatment. The plating and shake flask experiment described above was repeated, and one isolate was found by GC analysis to contain 11% poly(4-hydroxybutate) by weight. This strain was denoted MBX1476 and selected for further manipulations. The NTG treatment was repeated and killed 96.3% of the cells. The plating and shake flask experiment described above was repeated once again, and one isolate was found by GC analysis to contain 19% poly(4-hydroxybutate) by weight. This strain was denoted MBX1509.

### Example 15: PHBH Copolymer Producing Transgenic E. coli Strains.

*E coli* MBX240 is an XL1-blue (Stratagene, San Diego, CA) derivative with a chromosomally integrated copy of the PHB polymerase encoding *phbC* gene from *Ralstonia eutropha.* This strain does not form PHAs from carbon sources such as glucose or fatty acids, because of the absence of enzymes converting acetyl-CoA (generated from carbohydrates such as glucose) or fatty acid oxidation intermediates, into (R)-3-hydroxyacyl-CoA monomers for polymerization. pMSXJ12 was constructed by inserting the *phaJ* gene from *A. caviae* digested with EcoRI and PstI into the corresponding sites of pUC1BSfi. The *phaJ* gene was obtained by polymerase chain reaction using the primers Ac3-5': 5' AGAATTCAGGAGGACGCCGCATGAGCGCACAATCCCTGG and Ac3-3': 5' TTCCTGCAGCTCAAGGCAGCTTGACCACG using a PCR program including 30 cycles of 45 s at 95 C, 45 s at 55 C and 2.5 minutes at 72 C. Transformants of *E. coli* MBX240 with plasmid pMTXJ12 containing the (R)-specific enoyl-CoA hydratase encoded by the *phaJ* gene from *Aeromonas caviae* were grown on Luria-Bertani medium with 10 mM octanoate and 1 mM oleate. After 48 hours of growth, cells were harvested from a 50 ml culture by centrifugation and the cell pellet lyophilized. Lyophilized cells were extracted with chloroform (8 ml) for 16 hours and PHA was specifically precipitated from the chloroform solution by adding the chloroform layer to a 10-fold excess ethanol. Precipitation was allowed to occur at 4 C and the solid polymer was air dried and analyzed for composition by acidic butanolysis. Butylated PHA monomers were separated by gas chromatography and identified the PHA as a poly(3-hydroxybutyrate-co-3-hydroxyhexanoate) copolymer with 2.6 % 3-hydroxyhexanoate monomers.

### Example 16: Construction of Transgenic E. coli Strains for Screening of New and/or Improved Genes for PHA Production.

The *phbC* gene was introduced into an *E*. *coli* cloning strain by bacteriophage P1 transduction. In a procedure similar to that followed for *phbC₅* integration, the *phbC* gene from *R. eutropha* was integrated into the chromosome of MB_X23, resulting in MBX143. After chloramphenicol amplification, MBX150, which is resistant to 500 µg/ml chloramphenicol, was isolated. A bacteriophage P1 lysate grown on MBX150 was used to transduce the *phbC-cat* allele into XL1-Blue [pT7-RecA]. Plasmid pT7RecA expresses a functional RecA protein which is required for successful P1 transduction. The resulting strain MBX240 is an XL1-Blue derivative with a functional PHB polymerase expressed from the chromosome. MBX613 and MBX683 were developed using the same procedures. These strains were derived from MBX245 and XL1-Blue, respectively, and contain integrated AB₅cat (MBX613) or p₁₃AB₅kan (MBX683) operons.

### Example 17: Identification of Genes Encoding New, Improved, or Ancillary PHA Biosynthetic Enzymes.

MBX240, 613, and 683 are three strains that can be used in screening procedures for new or improved PHA genes. Using these strains, the following genes have been identified: *phbCABFA2* from *P*. *acidophila* and *phbCAB* from *A. latus.* In addition, the *phaJ* gene from *A. caviae* was functionally expressed in MBX240 to produce PHA from fatty acids. Besides PHA biosynthetic genes specific for C₃ to C₆ monomers, PHA biosynthetic enzymes for PHAs consisting of medium side-chain 3-hydroxy acids can also be expressed in *E. coli.* Such strains are useful in identifying additional PHA biosynthetic enzymes.

### Example 18: Integration of pha genes in R. eutropha.

The plasmids described in the previous examples were used to integrate *pha* genes in *R. eutropha.* Using a PHA-negative mutant of *R. eutropha* such as #2 (Peoples & Sinskey, J. Biol. Chem. 264:15298-303 (1989)) or PHB⁻4 (Schubert, et al., J. Bacteriol. 170:5837-47 (1988)), PHA formation was restored by integration of *phaC* from *A. caviae* in combination with *phbAB* from *Z*. *ramigera* or *phaJ* from *A. caviae.* The resulting strains produced PHAs to variable levels, with a molecular weight in the range of 400,000 to 10,000,000 Da and with a composition that includes monomers such as 3-hydroxyhexanoate and 3-hydroxyoctanoate.

### Example 19: Integration of pha Genes in P. putida.

The plasmids described in the previous examples were used to integrate *pha* genes into *Pseudomonas putida.* The PHA-negative phenotype of *P. putida* GPp104 (Huisman et al., J. Biol. Chem. 266:2191-98 (1991)) was restored by integration of a phaC3kan cassette where *phaC3* encodes the PHA polymerase from *P. oleovorans.* Integration of phaC3kan using pMUXC3kan was also applied to generate mutants of *P. putida* with mutations in genes encoding enzymes that affect PHA metabolism other than *phaC.* The PHA polymerase gene from *A. caviae* was also introduced in to the chromosome to result in a strain that produces PHAs including 3-hydroxy fatty acids in the C₃ to C₉ range.

### Example 20: Chromosomal Integration of phaC Genes to Control Molecular Weight of the Resulting PHA.

It is well known that the concentration of PHA polymerase determines the molecular weight of the produced PHA when substrate is available in excess. Variation of the molecular weight is desirable as polymer properties are dependent on molecular weight. Chromosomal integration of *phb* genes results in variable levels of expression of the *pha* gene as determined by the chromosomal integration site. It is therefore possible to obtain different transgenic bacteria that have variable levels of *phaC* expression and hence produce PHAs of variable molecular weight. With this system, it is possible to produce PHAs with molecular weights of greater than 400,000 Da and frequently even in excess of 1,000,000 Da. This procedure is applicable to any gram-negative bacterium in which the pUT or pLOF derived plasmids can be introduced, such as *E. coli*, *R. eutropha*, *P. putida, Klebsiella pneumoniae, Alcaligenes latus, Azotobacter vinelandii*, *Burkholderia cepacia*, *Paracoccus denitrificans* and in general in species of the *Escherichia*, *Pseudomonas*, *Ralstonia*, *Burkholderia*, *Alcaligenes*, *Klebsiella*, *Azotobacter* genera.

### Example 21: Integration of the PHB genes as a single operon.

A plasmid, pMSXABC₅kan, was constructed such that the thiolase (*phbA*), reductase (*phbB*), and PHB synthase (*phbC*) genes from *Zoogloea ramigera* and the kanamycin resistance gene (kan) were linked as an operon in the vector pUC18Sfi. This expression cassette was then excised as an *Avr*II fragment and inserted into the *Avr*II site of pUT to obtain pMUXABC₅kan.

S17-1 λpir strains with pMUXABC₅kan were mated with MBX247. Transgenic strains in which *phbABC*₅kan had integrated into the chromosome were selected on LB/Nl/Km plates. Among the integrants, PHB producers were identified on LB/glucose plates. One strain thus constructed, MBX1164, was selected for further study.

Thiolase (Nishimura et al., 1978, Arch. Microbiol. 116:21-24) and reductase (Saito et al., 1977, Arch. Microbiol. 114:211-217) assays were conducted on MBX1164 crude extracts. The cultures were grown in 50 mL of 0.5 x E2 medium supplemented with 20 g/L glucose. One unit (U) was defined as the amount of enzyme that converted 1 µmol of substrate to product per min. 3-Ketothiolase activity was determined to be 2.23 ± 0.38 and 2.48 ± 0.50 U/mg in two independent trials, and 3-hydroxybutyryl-CoA reductase activity was determined to be 4.10 ± 1.51 and 3.87 ± 0.15 U/mg in two independent trials.

Strain MBX1164 was evaluated for its PHB-producing ability in square shake bottles. The cells were grown in 2 mL of LB, and 0.1 mL of this was used as an inoculum for the 50-mL shake bottle culture. The shake bottle contained E2 medium supplemented with 0.25% corn steep liquor (Sigma, St. Louis, MO) and 20 g/L glucose. After incubation at 30°C for 48 hours with shaking at 200 rpm, the biomass concentration had reached 2.6 g/L, and the PHB concentration had reached 11.7 g/L; thus the cells contained 82% PHB by weight.

### Example 22: Integration of the Pseudomonas oleovorans PHA synthase into the E. coli chromosome.

A PHA synthase (*phaC*) cassette from the *P. oleovorans* chromosome and a promoterless chloramphenicol resistance gene were inserted into pUC118 such that an operon of the two genes was formed; i.e., they were oriented in the same direction and could be transcribed on the same mRNA. The sequence of the *P.oleovorans phaC* gene is shown below. The *phaC*-cat operon was excised from this plasmid by digestion with *Kpn*I and *Hind*III and ligated to pUC18SfiI that had been digested with the same two enzymes to form pMSXC₃cat. This allowed the *phaC-cat* operon to be flanked by *Avr*II sites. The *phaC*-cat operon was removed from pMSXC₃cat by digestion with *Avr*II and *Fsp*I. Because the two *Avr*II fragments of pMSXC₃cat were nearly the same size, *Fsp*I was used to facilitate isolation of the *phaC*-cat operon by cutting the rest of the vector into two pieces. The *Avr*II fragment was ligated to pUTkan which had been digested with *Avr*II and treated with alkaline phosphatase to prevent self-ligation. The plasmid thus produced was denoted pMUXC₃cat. The operon on this plasmid actually consisted of *phaC*-cat-kan. Strain CC118 λpir (a λpir lysogenic strain) was transformed with pMUXC₃cat to produce strain MBX130. Equal amounts of strains MBX130 and MBX245 were mixed on an LB agar plate and incubated for 8 hours at 37°C. The mixed cells were then used as an inoculum for an overnight 37°C culture of LB-chloramphenicol (25 µg/mL)-nalidixic acid (30 µg/mL). Single colonies were isolated from this culture by plating on LB-chloramphenicol (25 µg/ML)-nalidixic acid (30 µg/mL)-kanamycin (25 µg/mL). The colonies thus isolated have a transducible *phaC*-cat-kan cassette on the chromosome, as shown by the ability to use P1 transduction to introduce the cassette into the chromosome of other strains and select for resistance to both chloramphenicol and kanamycin.
*Pseudomonas oleovorans* PHA synthase (*phaC*).

## Claims

1. A genetically engineered microorganism having a PHA synthase gene involved in synthesis of polyhydroxyalkanoates, wherein the microorganism is transformed with a *phaJ* gene encoding enoyl-CoA hydratase, and either or both of the PHA synthase and *phaJ* genes are integrated into the chromosome of the microorganism, and the microorganism produces polyhydroxyalkanoate, wherein the microorganism is selected from the group consisting of *E. coli, Alcaligenes latus, Alcaligenese eutrophus, Azotobacter, Pseudomonas putida,* and *Ralstonia eutropha.*

2. The genetically engineered microorganism of Claim 1 wherein the *phaJ* gene is integrated into the chromosome of the bacterium.

3. The microorganism of Claim 2 wherein the *phaJ* gene is inserted using transposon mutagenesis.

4. The genetically engineered microorganism of Claim 1 wherein the *phaJ* gene is present within the microorganism on a plasmid.

5. The microorganism of any preceding claim, wherein the *phaJ* gene encodes the (R)-specific enoyl-CoA hydratase from *A. caviae* (J12).

6. The genetically engineered microorganism of any one of Claims 1 to 5 wherein the PHA synthase gene is a transgene.

7. The genetically engineered microorganism of any preceding claim wherein the PHA synthase gene is integrated into the chromosome of the bacterium.

8. The genetically engineered microorganism of any preceding claim wherein the PHA synthase gene is a PHB synthase gene.

9. The genetically engineered microorganism of any one of Claims 6 to 8 wherein the PHA synthase is PHB polymerase from *R. eutropha* (C1), PHA polymerase from *P. oleovorans* (C3), or PHB polymerase from *A*. *caviae* (C12).

10. The microorganism of any preceding claim comprising multiple genes involved in synthesis of polyhydroxyalkanoate wherein the genes are integrated operably linked as an operon.

11. The microorganism of Claim 1 wherein the PHA synthase and/or the *phaJ* gene is integrated operably linked under the control of a promoter.

12. The microorganism of Claim 1 wherein the PHA synthase and/or the *phaJ* gene is integrated operably linked with upstream activating sequences, an mRNA stabilizing sequence, or a selection marker.

13. The microorganism of Claim 1 wherein the PHA synthase and/or the *phaJ* gene is operably linked with promoter including a consensus *E. coli* pho box and -35 promoter region that is regulated by the phosphate concentration in the medium.

14. The microorganism of Claim 13 wherein the promoter is selected from the group consisting of promoter that induces expression under general stress conditions such as nutrient limitation, pH or heat shock, and administration of toxic chemicals.

15. The microorganism of Claim 1 wherein the PHA synthase and/or the *phaJ* gene is isolated or derived from a microorganism selected from the group consisting of *A*. *eutrophus, Aeromonas caviae, Zoogloea ramigera, Nocardia, Rhodococcus, Pseudomonas Sp. 61-3, Pseudomonas acidophila, Pseudomonas oleovarans, Chromobacterium violaceum,* and *Alcaligenes latus.*

16. The microorganism of Claim 1 wherein the PHA synthase and/or the *phaJ* gene is integrated as a single copy on the chromosome of the microorganism.

17. A genetically engineered microorganism of any preceding claim that is capable of producing PHA from fatty acids.

18. The genetically engineered microorganism of any preceding claim further having at least one gene involved in synthesis of polyhydroxyalkanoates selected from the group consisting of thiolase, reductase, and acyl-CoA transferase, integrated into the chromosome.

19. A method for producing polyhydroxyalkanoates comprising culturing genetically engineered micro-organisms as defined by any preceding claim, with appropriate substrate under conditions wherein the micro-organisms produce polyhydroxyalkanoate.

20. The method of Claim 19 wherein the substrate includes a fatty acid, such as octanoate and/or oleate.

21. Use of an isolated DNA molecule comprising a sequence encoding enoyl-CoA hydratase (*PhaJ*) to modify the type of substrate used in the production of PHA, or to enhance the level, or cause a shift in the type, of PHA produced, by a PHA-producing micro-organism by integration of the DNA sequence into the chromosome of the PHA-producing micro-organism compared to the level, or type, of PHA produced by the same micro-organism prior to the integration of the DNA molecule.

22. Use according to Claim 21 of an isolated DNA molecule comprising a sequence encoding enoyl-CoA hydratase (*PhaJ*) to cause a production of PHA from fatty acids.

## Patentansprüche

1. Genetisch veränderter Mikroorganismus mit einem PHA-Synthasegen, das an der Synthese von Polyhydroxyalkanoaten beteiligt ist, wobei der Mikroorganismus mit einem *phaJ*-Gen transformiert wird, das für Enoyl-CoA-Hydratase kodiert, sodass das PHA-Synthase- und/oder das *phaJ*-Gen in das Chromosom des Mikroorganismus integriert ist, und der Mikroorganismus Polyhydroxyalkanoat produziert, wobei der Mikroorganismus ausgewählt ist aus der Gruppe bestehend aus *E. coli, Alcaligenes latus, Alcaligenes eutrophus, Azotobacter, Pseudomonas putida* und *Ralstonia eutropha.*

2. Genetisch veränderter Mikroorganismus nach Anspruch 1, wobei das *phaJ*-Gen in das Chromosom des Bakteriums integriert ist.

3. Mikroorganismus nach Anspruch 2, wobei das *phaJ*-Gen unter Verwendung von Transposonmutagenese eingefügt ist.

4. Genetisch veränderter Mikroorganismus nach Anspruch 1, wobei das *phaJ*-Gen in dem Mikroorganismus auf einem Plasmid vorliegt.

5. Mikroorganismus nach einem der vorhergehenden Ansprüche, wobei das *phaJ*-Gen für die (R)-spezifische Enoyl-CoA-Hydratase aus *A. caviae* (J12) codiert.

6. Genetisch veränderter Mikroorganismus nach einem der Ansprüche 1 bis 5, wobei das PHA-Synthasegen ein Transgen ist.

7. Genetisch veränderter Mikroorganismus nach einem der vorhergehenden Ansprüche, wobei das PHA-Synthasegen in das Chromosom des Bakteriums integriert ist.

8. Genetisch veränderter Mikroorganismus nach einem der vorhergehenden Ansprüche, wobei das PHA-Synthasegen ein PHB-Synthasegen ist.

9. Genetisch veränderter Mikroorganismus nach einem der Ansprüche 6 bis 8, wobei die PHA-Synthase PHB-Polymerase von *R. eutropha* (C1), PHA-Polymerase von *P*. *oleovorans* (C3) oder PHB-Polymerase von *A. caviae* (C12) ist.

10. Mikroorganismus nach einem der vorhergehenden Ansprüche, umfassend mehrere Gene, die an der Synthese von Polyhydroxyalkanoaten beteiligt sind, wobei die Gene als ein Operon funktionsfähig verknüpft integriert sind.

11. Mikroorganismus nach Anspruch 1, wobei die PHA-Synthase und/oder das *phaJ-*Gen von einem Promoter gesteuert funktionsfähig verknüpft integriert ist.

12. Mikroorganismus nach Anspruch 1, wobei die PHA-Synthase und/oder das *phaJ-*Gen mit Upstream-Aktivierungssequenzen, einer mRNA-stabilisierenden Sequenz oder einem Selektionsmarker funktionsfähig verknüpft integriert ist.

13. Mikroorganismus nach Anspruch 1, wobei die PHA-Synthase und/oder das *phaJ-*Gen funktionsfähig mit einem Promoter verknüpft ist, einschließlich einer Konsensus-*E. coli-*Pho-Box und einer -35-Promoterregion, die durch die Phosphatkonzentration in dem Medium reguliert wird

14. Mikroorganismus nach Anspruch 13, wobei der Promoter ausgewählt ist aus der Gruppe bestehend aus Promoter, welcher Exprimierung unter allgemeinen Belastungsbedingungen induziert, wie etwa Nährstoffeinschränkung, pH- oder Wärmeschock und Hinzufügen von toxischen Chemikalien.

15. Mikroorganismus nach Anspruch 1, wobei die PHA-Synthase und/oder das *phaJ-*Gen aus einem Mikroorganismus, ausgewählt aus der Gruppe bestehend aus *A. eutrophus, Aeromonas caviae, Zoogloea ramigera, Nocardia, Rhodococcus, Pseudomonas Sp. 61-3, Pseudomonas acidophila, Pseudomonas oleovorans, Chromobacterium violaceum* und *Alcaligenes latus* isoliert oder abgeleitet ist.

16. Mikroorganismus nach Anspruch 1, wobei die PHA-Synthase und/oder das *phaJ-*Gen als eine Einzelkopie auf dem Chromosom des Mikroorganismus integriert ist.

17. Genetisch veränderter Mikroorganismus nach einem der vorhergehenden Ansprüche, der in der Lage ist, PHA aus Fettsäuren zu produzieren.

18. Genetisch veränderter Mikroorganismus nach einem der vorhergehenden Ansprüche, ferner wenigstens ein in das Chromosom integrierte Gen aufweisend, das an der Synthese von Polyhydroxyalkanoaten beteiligt ist, ausgewählt aus der Gruppe bestehend aus Thiolase, Reduktase und Acyl-CoA-Transferase.

19. Verfahren zum Produzieren von Polyhydroxyalkanoaten, umfassend das Kultivieren von genetisch veränderten Mikroorganismen nach einem der vorhergehenden Ansprüche mit einem geeigneten Substrat unter Bedingungen, in denen die Mikroorganismen Polyhydroxyalkanoat produzieren.

20. Verfahren nach Anspruch 19, wobei das Substrat eine Fettsäure enthält, wie etwa Octanoat und/oder Oleat.

21. Gebrauch eines isolierten DNA-Moleküls, umfassend eine Sequenz, die für Enoyl-CoA-Hydratase (*PhaJ*) codiert, um den Typ des bei der Produktion von PHA eingesetzten Substrats zu verändern oder zum Erhöhen der Menge oder zum Verursachen einer Veränderung des Typs des durch einen PHA produzierenden Mikroorganismus erzeugten PHA durch Integration der DNA-Sequenz in das Chromosom des PHA produzierenden Mikroorganismus, verglichen mit der Menge oder dem Typ von durch denselben Mikroorganismus produziertem PHA vor der Integration des DNA-Moleküls.

22. Gebrauch eines isolierten DNA-Moleküls nach Anspruch 21, umfassend eine Sequenz, die für Enoyl-CoA-Hydratase (*PhaJ*) codiert, um eine Produktion von PHA aus Fettsäuren zu bewirken.

## Revendications

1. Micro-organisme génétiquement modifié ayant un gène PHA synthase impliqué dans la synthèse de polyhydroxyalcanoates, lequel micro-organisme est transformé avec un gène *phaJ* codant pour l'énoyl-CoA hydratase, et l'un ou les deux de la PHA synthase et des gènes *phaJ* sont intégrés dans le chromosome du micro-organisme, et le micro-organisme produit du polyhydroxyalcanoate, où le micro-organisme est choisi dans le groupe constitué par *E coli, Alcaligenes latus, Alcaligenes eutrophus, Azobacter, Pseudomonas putida* et *Ralstonia eutropha.*

2. Micro-organisme génétiquement modifié selon la revendication 1, dans lequel le gène *phaJ* est intégré dans le chromosome de la bactérie.

3. Micro-organisme selon la revendication 2, dans lequel le gène *phaJ* est inséré par mutagenèse par transposon.

4. Micro-organisme génétiquement modifié selon la revendication 1, dans lequel le gène *phaJ* est présent dans le micro-organisme sur un plasmide.

5. Micro-organisme selon l'une quelconque des revendications précédentes, dans lequel le gène *phaJ* code pour l'énoyl-CoA hydratase (R)-spécifique de *A. caviae* (J12).

6. Micro-organisme génétiquement modifié selon l'une quelconque des revendications 1 à 5, dans lequel le gène PHA synthase est un transgène.

7. Micro-organisme génétiquement modifié selon l'une quelconque des revendications précédentes, dans lequel le gène PHA synthase est intégré dans le chromosome de la bactérie.

8. Micro-organisme génétiquement modifié selon l'une quelconque des revendications précédentes, dans lequel le gène PHA synthase est un gène PHB synthase.

9. Micro-organisme génétiquement modifié selon l'une quelconque des revendications 6 à 8, dans lequel le gène PHA synthase est une PHB polymérase de *R*. *eutropha* (C1), une PHA polymérase de *P*. *oleovorans* (C3) ou une PHB polymérase de *A. caviae* (C12).

10. Micro-organisme selon l'une quelconque des revendications précédentes comprenant plusieurs gènes impliqués dans la synthèse de polyhydroxyalcanoate, dans lequel les gènes sont intégrés fonctionnellement liés sous la forme d'un opéron.

11. Micro-organisme selon la revendication 1, dans lequel la PHA synthase et/ou le gène *phaJ* sont intégrés fonctionnellement liés sous le contrôle d'un promoteur.

12. Micro-organisme selon la revendication 1, dans lequel la PHA synthase et/ou le gène *phaJ* sont intégrés fonctionnellement liés aux séquences activatrices en amont, une séquence stabilisatrice d'ARNm ou un marqueur de sélection.

13. Micro-organisme selon la revendication 1, dans lequel la PHA synthase et/ou le gène *phaJ* sont fonctionnellement liés au promoteur incluant une boîte pho d'*E. coli* consensus et une région promotrice -35 qui est régulée par la concentration en phosphate dans le milieu.

14. Micro-organisme selon la revendication 13, dans lequel le promoteur est choisi dans le groupe constitué par le promoteur qui induit l'expression sous des conditions de stress général telles que l'oligotrophie, le pH ou le choc thermique et l'administration de substances chimiques toxiques.

15. Micro-organisme selon la revendication 1, dans lequel la PHA synthase et/ou le gène *phaJ* sont isolés ou extraits d'un micro-organisme choisi dans le groupe constitué par *A*. *eutrophus, Aeromonas caviae, Zoogloea ramigera, Nocardia, Rhodococcus, Pseudomonas, Sp. 61*-*3*, *Pseudomonas acidophila, Pseudomonas oleovorans, Chromobacterium violaceum* et *Alcaligenes latus.*

16. Micro-organisme selon la revendication 1, dans lequel la PHA synthase et/ou le gène *phaJ* sont intégrés sous la forme d'une seule copie sur le chromosome du micro-organisme.

17. Micro-organisme génétiquement modifié selon l'une quelconque des revendications précédentes, qui est capable de produire du PHA à partir d'acides gras.

18. Micro-organisme génétiquement modifié selon l'une quelconque des revendications précédentes, ayant en outre au moins un gène impliqué dans la synthèse de polyhydroxyalcanoates choisi dans le groupe constitué par la thiolase, la réductase et l'acyl-CoA transférase, intégré dans le chromosome.

19. Procédé de production de polyhydroxyalcanoates comprenant la culture de micro-organismes génétiquement modifiés tels que définis par l'une quelconque des revendications précédentes, avec un substrat approprié dans des conditions dans lesquelles les micro-organismes produisent du polyhydroxyalcanoate.

20. Procédé selon la revendication 19, dans lequel le substrat comprend un acide gras tel que l'octanoate et/ou l'oléate.

21. Utilisation d'une molécule d'ADN isolée comprenant une séquence codant pour l'énoyl-CoA hydratase (*PhaJ*) pour modifier le type de substrat utilisé dans la production de PHA, ou pour accroître le niveau, ou causer un réarrangement, du type de PHA produit, par un micro-organisme producteur de PHA par l'intégration de la séquence d'ADN dans le chromosome du micro-organisme producteur de PHA par comparaison au niveau, ou type, de PHA produit par le même micro-organisme avant l'intégration de la molécule d'ADN.

22. Utilisation selon la revendication 21 d'une molécule d'ADN isolée comprenant une séquence codant pour l'énoyl-CoA hydratase (*PhaJ*) pour provoquer une production de PHA à partir d'acides gras.
